# EUROPEAN PATENT SPECIFICATION

(11) **EP 2 365 004 B1**
(45) Date of publication and mention of the grant of the patent: **06.01.2016**
(21) Application number: 11159411.5
(22) Date of filing: 23.06.2003
(51) Int. Cl.: C07K 16/28, C07K 14/705, A61K 39/395, A61K 38/17, G01N 33/68, C12Q 1/68

(54) **Membrane associated tumor endothelium markers**
Membranassoziierte Tumorendothel-Marker
Marqueurs associés à la membrane d'endothélium tumoral

(30) Priority: 21.06.2002 US 390187 P; 01.04.2003 US 458959 P
(43) Date of publication of application: 14.09.2011
(62) Divisional of application: 03742108.8
(73) Proprietor: Johns Hopkins University School of Medicine, Baltimore, MD 21201 (US)
(72) Inventor: St. Croix, Brad, Cockeysville, MD 21030.5837 (US); Kinzler, Kenneth, Bel Air, MD 21015 (US); Vogelstein, Bert, Baltimore, MD 21208 (US)
(74) Representative: Tombling, Adrian George

(56) References cited:
- EP-A1- 0 953 639
- WO-A1-94/11023
- WO-A2-02/10217
- DATABASE MEDLINE [Online] US NATIONAL LIBRARY OF MEDICINE (NLM), BETHESDA, MD, US NLM22236832, 01 February 2012 LIU RUI ET AL: 'Fibroblast activation protein: A potential therapeutic target in cancer.' Database accession no. NLM22236832
- DATABASE MEDLINE [Online] US NATIONAL LIBRARY OF MEDICINE (NLM), BETHESDA, MD, US NLM19920354, December 2009 SANTOS ANGÉLICA M ET AL: 'Targeting fibroblast activation protein inhibits tumor stromagenesis and growth in mice.' Database accession no. NLM19920354
- DATABASE MEDLINE [Online] US NATIONAL LIBRARY OF MEDICINE (NLM), BETHESDA, MD, US NLM15939342, February 2005 KELLY THOMAS: 'Fibroblast activation protein-alpha and dipeptidyl peptidase IV (CD26): cell-surface proteases that activate cell signaling and are potential targets for cancer therapy.' Database accession no. NLM15939342
- DATABASE MEDLINE [Online] US NATIONAL LIBRARY OF MEDICINE (NLM), BETHESDA, MD, US NLM19606930, August 2009 PURÉ ELLEN: 'The road to integrative cancer therapies: emergence of a tumor-associated fibroblast protease as a potential therapeutic target in cancer.' Database accession no. NLM19606930

## Description

### TECHNICAL FIELD OF THE INVENTION

This invention is related to the area of angiogenesis and anti-angiogenesis. In particular, it relates to a method for detecting genes which are characteristically expressed in tumor endothelial and normal endothelial cells.

### BACKGROUND OF THE INVENTION

It is now widely recognized that tumors require a blood supply for expansive growth. This recognition has stimulated a profusion of research on tumor angiogenesis, based on the idea that the vasculature in tumors represents a potential therapeutic target. However, several basic questions about tumor endothelium remain unanswered. For example, are vessels of tumors qualitatively different from normal vessels of the same tissue? What is the relationship of tumor endothelium to endothelium of healing wounds or other physiological or pathological forms of angiogenesis? The answers to these questions critically impact on the potential for new therapeutic approaches to inhibit angiogenesis in a specific manner.

There is a continuing need in the art to characterize the vasculature of tumors relative to normal vasculature so that any differences can be exploited for therapeutic and diagnostic benefits.

One technique which can be used to characterize gene expression, or more precisely gene transcription, is termed serial analysis of gene expression (SAGE). Briefly, the SAGE approach is a method for the rapid quantitative and qualitative analysis of mRNA transcripts based upon the isolation and analysis of short defined sequence tags (SAGE Tags) corresponding to expressed genes. Each Tag is a short nucleotide sequences (9-17 base pairs in length) from a defined position in the transcript. In the SAGE method, the Tags are dimerized to reduce bias inherent in cloning or amplification reactions. (See, US Patent 5,695,937.) SAGE is particularly suited to the characterization of genes associated with vasculature stimulation or inhibition because it is capable of detecting rare sequences, evaluating large numbers of sequences at one time, and to provide a basis for the identification of previously unknown genes.

WO02/10217 identifies numerous genes differentially expressed in tumor and normal endothelial cells. However, there is no disclosure of FAP being differentially expressed.

### SUMMARY OF THE INVENTION

The invention provides a method for detecting tumor endothelium in a patent in accordance with claim 1.

This and other embodiments which will be apparent to those of skill in the art upon reading the specification provide the art with reagents and methods for detection, diagnosis, therapy, and drug screening pertaining to neoangiogenesis and pathological processes involving or requiring neoangiogenesis.

### DETAILED DESCRIPTION OF THE INVENTION

We identified 76 human genes that are expressed at significantly higher levels (≥ 2-fold) in tumor endothelium than in normal endothelium and that encode membrane proteins. See Table 1. Most of these genes were either not expressed or expressed at relatively low levels in Endothelial Cells (ECs) maintained in culture. Interestingly, the tumor endothelium genes were expressed in all tumors tested, regardless of its tissue or organ source. Most tumor endothelium genes were also expressed in corpus luteum and wounds.

It is clear that normal and tumor endothelium are highly related, sharing many endothelial cell specific markers. It is equally clear that the endothelium derived from tumors is qualitatively different from that derived from normal tissues of the same type and is also different from primary endothelial cultures. These genes are characteristically expressed in tumors derived from several different tissue types, documenting that tumor endothelium, in general, is different from normal endothelium. The genes expressed differentially in tumor endothelium are also expressed during other angiogenic processes such as corpus luteum formation and wound healing. It is therefore more appropriate to regard the formation of new vessels in tumors as "neoangiogenesis" rather than "tumor angiogenesis" *per se.* This distinction is important from a variety of perspectives, and is consistent with the idea that tumors recruit vasculature using much of, or basically the same signals elaborated during other physiologic or pathological processes. That tumors represent "unhealed wounds" is one of the oldest ideas in cancer biology.

Sequence and literature study has permitted the following identifications to be made among the family of TEM proteins. Membrane associated TEM proteins have been identified which contain transmembrane regions. These include potassium inwardly-rectifying channel, subfamily J, member 8; vascular cell adhesion molecule 1; NADH:ubiquinone oxidoreductase MLRQ subunit homolog; hypothetical protein MGC5508; syndecan 2 (heparan sulfate proteoglycan 1, cell surface-associated, fibroglycan); hypothetical protein BC002942; uncharacterized hematopoietic; stem/progenitor cells protein MDS032; FAT tumor suppressor homolog 1 (Drosophila); G protein-coupled receptor 4; amyloid beta (A4) precursor protein (protease nexin-II, Alzheimer disease); tumor necrosis factor receptor superfamily, member 25 (translocating chain-association membrane protein); major histocompatibility complex, class I, A; degenerative spermatocyte homolog, lipid desaturase (Drosophila); matrix metalloproteinase 25; prostate stem cell antigen; melanoma cell; adhesion molecule; G protein-coupled receptor; protocadherin beta 9; matrix; metalloproteinase 14 (membrane-inserted); scotin; chemokine (C-X-C motif) ligand 14; murine retrovirus integration site 1 homolog; integrin, alpha 11; interferon, alpha-; inducible protein (clone IFI-6-16); CLST 11240 protein; H factor (complement)-like; tweety homolog 2 (Drosophila); transient receptor potential; cation channel, subfamily V, member 2; hypothetical protein PRO1855; sprouty homolog 4 (Drosophila); accessory protein BAP31; integrin, alpha V (vitronectin receptor, alpha polypeptide, antigen CD51); gap junction protein, alpha 4, 37kDa (connexin 37); calsyntenin 1; solute carrier family 26, member 6; family with sequence similarity 3, member C; immunoglobulin heavy constant gamma 3 (G3m marker); hephaestin; hypothetical protein DKFZp761D0211; cisplatin resistance related protein CRR9p; hypothetical protein IMAGE3455200; Homo sapiens mRNA full length insert cDNA clone EUROIMAGE881791; hypothetical protein MGC15523; prostaglandin I2 (prostacyclin) receptor (IP); CD164 antigen, sialomucin; putative G-protein coupled receptor GPCR41; DKFZP566H073 protein; platelet-derived growth factor receptor, alpha polypeptide; NADH dehydrogenase (ubiquinone) 1 alpha subcomplex, 1, 7.5kDa; CD151 antigen; platelet-derived growth factor receptor, beta polypeptide; KIAA0102 gene product; B7 homolog 3; solute carrier family 4, anion exchanger, member 2 (erythrocyte membrane protein band 3-like 1); endothelin receptor type B; defender against cell death 1; transmembrane, prostate androgen induced RNA; Notch homolog 3 (Drosophila); lymphotoxin beta (TNF superfamily, member 3) chondroitin sulfate proteoglycan 4 (melanoma-associated); lipoma HMGIC fusion partner; hypothetical protein similar to ankyrin repeat-containing protein AKR1; SDR1 short-chain dehydrogenase/reductase 1; PCSK7 proprotein convertase subtilisin/kexin type 7; Homo sapiens mRNA, cDNA DKFZp686D0720 (from clone DKFZp686D0720); FAP fibroblast activation protein, alpha; MCAM melanoma cell adhesion molecule; and CRELD1 cysteine-rich with EGF-like domains 1.

ECs represent only a minor fraction of the total cells within normal or tumor tissues, and only those EC transcripts expressed at the highest levels would be expected to be represented in libraries constructed from unfractionated tissues. The genes described in the current study should therefore provide a valuable resource for basic and clinical studies of human angiogenesis in the future. Nucleic acids and/or proteins corresponding to each of these genes are identified in Unigene, OMIM, and/or protein databases as indicated in Table 1.

Isolated and purified nucleic acids, are those which are not linked to those genes to which they are linked in the human genome. Moreover, they are not present in a mixture such as a library containing a multitude of distinct sequences from distinct genes. They may be, however, linked to other genes such as vector sequences or sequences of other genes to which they are not naturally adjacent. Tags disclosed herein, because of the way that they were made, represent sequences which are 3' of the 3' most restriction enzyme recognition site for the tagging enzyme used to generate the SAGE tags. In this case, the tags are 3' of the most 3' most NIaIII site in the cDNA molecules corresponding to mRNA. Nucleic acids corresponding to tags may be RNA, cDNA, or genomic DNA, for example. Such corresponding nucleic acids can be determined by comparison to sequence databases to determine sequence identities. Sequence comparisons can be done using any available technique, such as BLAST, available from the National Library of Medicine, National Center for Biotechnology Information. Tags can also be used as hybridization probes to libraries of genomic or cDNA to identify the genes from which they derive. Thus, using sequence comparisons or cloning, or combinations of these methods, one skilled in the art can obtain full-length nucleic acid sequences. Genes corresponding to tags will contain the sequence of the tag at the 3' end of the coding sequence or of the 3' untranslated region (UTR), 3' of the 3' most recognition site in the cDNA for the restriction endonuclease which was used to make the tags. The nucleic acids may represent either the sense or the anti-sense strand. Nucleic acids and proteins although disclosed herein with sequence particularity, may be derived from a single individual. Allelic variants which occur in the population of humans are included within the scope of such nucleic acids and proteins. Those of skill in the art are well able to identify allelic variants as being the same gene or protein. Given a nucleic acid, one of ordinary skill in the art can readily determine an open reading frame present, and consequently the sequence of a polypeptide encoded by the open reading frame and, using techniques well known in the art, express such protein in a suitable host. Proteins comprising such polypeptides can be the naturally occurring proteins, fusion proteins comprising exogenous sequences from other genes from humans or other species, epitope tagged polypeptides, etc. Isolated and purified proteins are not in a cell, and are separated from the normal cellular constituents, such as nucleic acids, lipids, etc. Typically the protein is purified to such an extent that it comprises the predominant species of protein in the composition, such as greater than 50, 60 70, 80, 90, or even 95% of the proteins present.

Using the proteins, one of ordinary skill in the art can readily generate antibodies which specifically bind to the proteins. Such antibodies can be monoclonal or polyclonal. They can be chimeric, humanized, or totally human. Any functional fragment or derivative of an antibody can be used including Fab, Fab', Fab2, Fab'2, and single chain variable regions. So long as the fragment or derivative retains specificity of binding for the endothelial marker protein it can be used. Antibodies can be tested for specificity of binding by comparing binding to appropriate antigen to binding to irrelevant antigen or antigen mixture under a given set of conditions. If the antibody binds to the appropriate antigen at least 2, 5, 7, and preferably 10 times more than to irrelevant antigen or antigen mixture then it is considered to be specific.

Techniques for making such partially to fully human antibodies are known in the art and any such techniques can be used. , Fully human antibody sequences can be made in a transgenic mouse which has been engineered to express human heavy and light chain antibody genes. Multiple strains of such transgenic mice have been made which can produce different classes of antibodies. B cells from transgenic mice which are producing a desirable antibody can be fused to make hybridoma cell lines for continuous production of the desired antibody. See for example, Nina D. Russel, Jose R. F. Corvalan, Michael L. Gallo, C. Geoffrey Davis, Liise-Anne Pirofski. Production of Protective Human Antipneumococcal Antibodies by Transgenic Mice with Human Immunoglobulin Loci Injection and Immunity April 2000, p. 1820-1826; Michael L. Gallo, Vladimir E. Ivanov, Aya Jakobovits, and C. Geoffrey Davis. The human immunoglobulin loci introduced into mice: V (D) and J gene segment usage similar to that of adult humans European Journal of Immunology 30: 534-540, 2000; Larry L. Green. Antibody engineering via genetic engineering of the mouse: XenoMouse strains are a vehicle for the facile generation of therapeutic human monoclonal antibodies Journal of Immunological Methods 231 11-23, 1999; Yang X-D, Corvalan JRF, Wang P, Roy CM-N and Davis CG. Fully Human Anti-interleukin-8 Monoclonal Antibodies: Potential Therapeutics for the Treatment of Inflammatory Disease States. Journal of Leukocyte Biology Vol. 66, pp401-410 (1999); Yang X-D, Jia X-C, Corvalan JRF, Wang P, CG Davis and Jakobovits A. Eradication of Established Tumors by a Fully Human Monoclonal Antibody to the Epidermal Growth Factor Receptor without Concomitant Chemotherapy. Cancer Research Vol. 59, Number 6, pp1236-1243 (1999) ; Jakobovits A. Production and selection of antigen-specific fully human monoclonal antibodies from mice engineered with human Ig loci. Advanced Drug Delivery Reviews Vol. 31, pp: 33-42 (1998); Green L and Jakobovits A. Regulation of B cell development by variable gene complexity in mice reconstituted with human immunoglobulin yeast artificial chromosomes. J. Exp. Med. Vol. 188, Number 3, pp: 483-495 (1998); Jakobovits A. The long-awaited magic bullets: therapeutic human monoclonal antibodies from transgenic mice. Exp. Opin. Invest. Drugs Vol. 7(4), pp : 607-614 (1998); Tsuda H, Maynard-Currie K, Reid L, Yoshida T, Edamura K, Maeda N, Smithies O, Jakobovits A. Inactivation of Mouse HPRT locus by a 203-bp retrotransposon insertion and a 55-kb gene-targeted deletion: establishment of new HPRT-Deficient mouse embryonic stem cell lines. Genomics Vol. 42, pp: 413-421 (1997); Sherman-Gold, R. Monoclonal Antibodies: The Evolution from '80s Magic Bullets To Mature, Mainstream Applications as Clinical Therapeutics. Genetic Engineering News Vol. 17, Number 14 (August 1997); Mendez M, Green L, Corvalan J, Jia X-C, Maynard-Currie C, Yang X-d, Gallo M, Louie D, Lee D, Erickson K, Luna J, Roy C, Abderrahim H, Kirschenbaum F, Noguchi M, Smith D, Fukushima A, Hales J, Finer M, Davis C, Zsebo K, Jakobovits A. Functional transplant of megabase human immunoglobulin loci recapitulates human antibody response in mice. Nature Genetics Vol. 15, pp: 146-156 (1997); Jakobovits A. Mice engineered with human immunoglobulin YACs: A new technology for production of fully human antibodies for autoimmunity therapy. Weir's Handbook of Experimental Immunology, The Integrated Immune System Vol. IV, pp: 194.1-194.7 (1996) ; Jakobovits A. Production of fully human antibodies by transgenic mice. Current Opinion in Biotechnology Vol. 6, No. 5, pp: 561-566 (1995) ; Mendez M, Abderrahim H, Noguchi M, David N, Hardy M, Green L, Tsuda H, Yoast S, Maynard-Currie C, Garza D, Gemmill R, Jakobovits A, Klapholz S. Analysis of the structural integrity of YACs comprising human immunoglobulin genes in yeast and in embryonic stem cells. Genomics Vol. 26, pp: 294-307 (1995); Jakobovits A. YAC Vectors: Humanizing the mouse genome. Current Biology Vol. 4, No. 8, pp: 761-763 (1994); Arbones M, Ord D, Ley K, Ratech H, Maynard-Curry K, Otten G, Capon D, Tedder T. Lymphocyte homing and leukocyte rolling and migration are impaired in L-selectin-deficient mice. Immunity Vol. 1, No. 4, pp: 247-260 (1994); Green L, Hardy M, Maynard-Curry K, Tsuda H, Louie D, Mendez M, Abderrahim H, Noguchi M, Smith D, Zeng Y, et. al. Antigen-specific human monoclonal antibodies from mice engineered with human Ig heavy and light chain YACs. Nature Genetics Vol. 7, No. 1, pp: 13-21 (1994); Jakobovits A, Moore A, Green L, Vergara G, Maynard-Curry K, Austin H, Klapholz S. Germ-line transmission and expression of a human-derived yeast artificial chromosome. Nature Vol. 362, No. 6417, pp: 255-258 (1993); Jakobovits A, Vergara G, Kennedy J, Hales J, McGuinness R, Casentini-Borocz D, Brenner D, Otten G. Analysis of homozygous mutant chimeric mice: deletion of the immunoglobulin heavy-chain joining region blocks B-cell development and antibody production. Proceedings of the National Academy of Sciences USA Vol. 90, No. 6, pp: 2551-2555 (1993); Kucherlapati et al., U.S. 6,1075,181.

Antibodies can also be made using phage display techniques. Such techniques can be used to isolate an initial antibody or to generate variants with altered specificity or avidity characteristics. Single chain Fv can also be used as is convenient. They can be made from vaccinated transgenic mice, if desired. Antibodies can be produced in cell culture, in phage, or in various animals, including but not limited to cows, rabbits, goats, mice, rats, hamsters, guinea pigs, sheep, dogs, cats, monkeys, chimpanzees, apes.

Antibodies can be labeled with a detectable moiety such as a radioactive atom, a chromophore, a fluorophore, or the like. Such labeled antibodies can be used for diagnostic techniques, either *in vivo,* or in an isolated test sample. Antibodies can also be conjugated, for example, to a pharmaceutical agent, such as chemotherapeutic drug or a toxin. They can be linked to a cytokine, to a ligand, to another antibody. Suitable agents for coupling to antibodies to achieve an anti-tumor effect include cytokines, such as interleukin 2 (IL-2) and Tumor Necrosis Factor (TNF); photosensitizers, for use in photodynamic therapy, including aluminum (III) phthalocyanine tetrasulfonate, hematoporphyrin, and phthalocyanine; radionuclides, such as iodine-131 (¹³¹I), yttrium-90 (⁹⁰Y), bismuth-212 (²¹²Bi), bismuth-213 (²¹³Bi), technetium-99m (^{99m}Tc), rhenium-186 (¹⁸⁶Re), and rhenium-188 (¹⁸⁸Re); antibiotics, such as doxorubicin, adriamycin, daunorubicin, methotrexate, daunomycin, neocarzinostatin, and carboplatin; bacterial, plant, and other toxins, such as diphtheria toxin, pseudomonas exotoxin A, staphylococcal enterotoxin A, abrin-A toxin, ricin A (deglycosylated ricin A and native ricin A), TGF-alpha toxin, cytotoxin from chinese cobra (naja naja atra), and gelonin (a plant toxin); ribosome inactivating proteins from plants, bacteria and fungi, such as restrictocin (a ribosome inactivating protein produced by *Aspergillus restrictus*), saporin (a ribosome inactivating protein from *Saponaria officinalis*), and RNase; tyrosine kinase inhibitors; ly207702 (a difluorinated purine nucleoside); liposomes containing antitumor agents (e.g., antisense oligonucleotides, plasmids which encode for toxins, methotrexate, etc.); and other antibodies or antibody fragments, such as F(ab).

Those of skill in the art will readily understand and be able to make such antibody derivatives, as they are well known in the art. The antibodies may be cytotoxic on their own, or they may be used to deliver cytotoxic agents to particular locations in the body. The antibodies can be administered to individuals in need thereof as a form of passive immunization.

Characterization of extracellular regions for the cell surface and secreted proteins from the protein sequence is based on the prediction of signal sequence, transmembrane domains and functional domains. Antibodies are preferably specifically immunoreactive with membrane associated proteins, particularly to extracellular domains of such proteins or to secreted proteins. Such targets are readily accessible to antibodies, which typically do not have access to the interior of cells or nuclei. However, in some applications, antibodies directed to intracellular proteins or epitopes may be useful as well. Moreover, for diagnostic purposes, an intracellular protein or epitope may be an equally good target since cell lysates may be used rather than a whole cell assay.

Computer programs can be used to identify extracellular domains of proteins whose sequences are known. Such programs include SMART software (Schultz et al., Proc. Natl. Acad. Sci. USA 95: 5857-5864,1998) and Pfam software (Bateman et al., Nucleic acids Res. 28: 263-266, 2000) as well as PSORTII. Typically such programs identify transmembrane domains; the extracellular domains are identified as immediately adjacent to the transmembrane domains. Prediction of extracellular regions and the signal cleavage sites are only approximate. It may have a margin of error + or - 5 residues. Signal sequence can be predicted using three different methods (Nielsen et al, Protein Engineering 10: 1-6 ,1997, Jagla et. al, Bioinformatics 16: 245-250,2000, Nakai, K and Horton, P. Trends in Biochem. Sci. 24:34-35, 1999) for greater accuracy. Similarly transmembrane (TM) domains can be identified by multiple prediction methods. (Pasquier, et. al, Protein Eng. 12:381-385, 1999, Sonnhammer et al., In Proc. of Sixth Int. Conf. on Intelligent Systems for Molecular Biology, p. 175-182, Ed J. Glasgow, T. Littlejohn, F. Major, R. Lathrop, D. Sankoff, and C. Sensen Menlo Park, CA: AAAI Press, 1998 , Klein, et.al, Biochim. Biophys. Acta, 815:468, 1985, Nakai and Kanehisa Genomics, 14: 897-911 , 1992). In ambiguous cases, locations of functional domains in well characterized proteins are used as a guide to assign a cellular localization.

Putative functions or functional domains of novel proteins can be inferred from homologous regions in the database identified by BLAST searches (Altschul et. al. Nucleic Acid Res. 25: 3389-3402, 1997) and/or from a conserved domain database such as Pfam (Bateman et.al, Nucleic Acids Res. 27:260-262 1999) BLOCKS (Henikoff, et. al, Nucl. Acids Res. 28:228-230, 2000) and SMART (Ponting, et. al, Nucleic Acid Res. 27,229-232, 1999). Extracellular domains include regions adjacent to a transmembrane domain in a single transmembrane domain protein (out-in or type I class). For multiple transmembrane domains proteins, the extracellular domain also includes those regions between two adjacent transmembrane domains (in-out and out-in). For type II transmembrane domain proteins, for which the N-terminal region is cytoplasmic, regions following the transmembrane domain is generally extracellular. Secreted proteins on the other hand do not have a transmembrane domain and hence the whole protein is considered as extracellular.

Membrane associated proteins can be engineered using standard techniques to delete the transmembrane domains, thus leaving the extracellular portions which can bind to ligands. Such soluble forms of transmembrane receptor proteins can be used to compete with natural forms for binding to ligand. Thus such soluble forms act as inhibitors. and can be used therapeutically as anti-angiogenic agents, as diagnostic tools for the quantification of natural ligands, and in assays for the identification of small molecules which modulate or mimic the activity of a TEM:ligand complex.

Alternatively, the endothelial markers themselves can be used as vaccines to raise an immune response in the vaccinated animal or human. For such uses, a protein, or immunogenic fragment of such protein, corresponding to the intracellular, extracellular or secreted TEM of interest is administered to a subject. The immogenic agent may be provided as a purified preparation or in an appropriately expressing cell. The administration may be direct, by the delivery of the immunogenic agent to the subject, or indirect, through the delivery of a nucleic acid encoding the immunogenic agent under conditions resulting in the expression of the immunogenic agent of interest in the subject. The TEM of interest may be delivered in an expressing cell, such as a purified population of tumor endothelial cells or a populations of fused tumor endothelial and dendritic cells. Nucleic acids encoding the TEM of interest may be delivered in a viral or non-viral delivery vector or vehicle. Non-human sequences encoding the human TEM of interest or other mammalian homolog can be used to induce the desired immunologic response in a human subject. For several TEMs, mouse, rat or other ortholog sequences can be obtained from the literature or using techniques well within the skill of the art.

Endothelial cells can be identified using the markers which are disclosed herein as being endothelial cell specific. These include the 76 human markers identified herein, *i.e.,* the tumor endothelial markers. Antibodies specific for such markers can be used to identify such cells, by contacting the antibodies with a population of cells containing some endothelial cells. The presence of cross-reactive material with the antibodies identifies particular cells as endothelial. Similarly, lysates of cells can be tested for the presence of cross-reactive material. Any known format or technique for detecting cross-reactive material can be used including, immunoblots, radioimmunoassay, ELISA, immunoprecipitation, and immunohistochemistry. In addition, nucleic acid probes for these markers can also be used to identify endothelial cells. Any hybridization technique known in the art including Northern blotting, RT-PCR, microarray hybridization, and in situ hybridization can be used.

One can identify tumor endothelial cells for diagnostic purposes, testing cells suspected of containing one or more TEMs. One can test both tissues and bodily fluids of a subject. For example, one can test a patient's blood for evidence of intracellular and membrane associated TEMs, as well as for secreted TEMs. Intracellular and/or membrane associated TEMs may be present in bodily fluids as the result of high levels of expression of these factors and/or through lysis of cells expressing the TEMs.

Populations of various types of endothelial cells can also be made using the antibodies to endothelial markers of the invention. The antibodies can be used to purify cell populations according to any technique known in the art, including but not limited to fluorescence activated cell sorting. Such techniques permit the isolation of populations which are at least 50, 60, 70, 80, 90, 92, 94, 95, 96, 97, 98, and even 99 % the type of endothelial cell desired, whether normal, tumor, or pan-endothelial. Antibodies can be used to both positively select and negatively select such populations. Preferably at least 1, 5, 10, 15, 20, or 25 of the appropriate markers are expressed by the endothelial cell population.

Populations of endothelial cells made as described herein, can be used for screening drugs to identify those suitable for inhibiting the growth of tumors by virtue of inhibiting the growth of the tumor vasculature.

Populations of endothelial cells made as described herein, can be used for screening candidate drugs to identify those suitable for modulating angiogenesis, such as for inhibiting the growth of tumors by virtue of inhibiting the growth of endothelial cells, such as inhibiting the growth of the tumor or other undesired vasculature, or alternatively, to promote the growth of endothelial cells and thus stimulate the growth of new or additional large vessel or microvasculature.

Inhibiting the growth of endothelial cells means either regression of vasculature which is already present, or the slowing or the absence of the development of new vascularization in a treated system as compared with a control system. By stimulating the growth of endothelial cells, one can influence development of new (neovascularization) or additional vasculature development (revascularization). A variety of model screen systems are available in which to test the angiogenic and/or anti-angiogenic properties of a given candidate drug. Typical tests involve assays measuring the endothelial cell response, such as proliferation, migration, differentiation and/or intracellular interaction of a given candidate drug. By such tests, one can study the signals and effects of the test stimuli. Some common screens involve measurement of the inhibition of heparanase, endothelial tube formation on Matrigel, scratch induced motility of endothelial cells, platelet-derived growth factor driven proliferation of vascular smooth muscle cells, and the rat aortic ring assay (which provides an advantage of capillary formation rather than just one cell type).

Drugs can be screened for the ability to mimic or modulate, inhibit or stimulate, growth of tumor endothelium cells and/or normal endothelial cells. Drugs can be screened for the ability to inhibit tumor endothelium growth but not normal endothelium growth or survival. Similarly, human cell populations, such as normal endothelium populations or tumor endothelial cell populations, can be contacted with test substances and the expression of tumor endothelial markers determined. Test substances which decrease the expression of tumor endothelial markers (TEMs) are candidates for inhibiting angiogenesis and the growth of tumors. In cases where the activity of a TEM is known, agents can be screened for their ability to decrease or increase the activity.

Drug candidates capable of binding to TEM receptors found at the cell surface can be identified. For some applications, the identification of drug candidates capable of blocking the TEM receptor from its native ligand will be desired. For some applications, the identification of a drug candidate capable of binding to the TEM receptor may be used as a means to deliver a therapeutic or diagnostic agent. For other applications, the identification of drug candidates capable of mimicking the activity of the native ligand will be desired. Thus, by manipulating the binding of a transmembrane TEM receptor: ligand complex, one may be able to promote or inhibit further development of endothelial cells and hence, vascularization.

Expression can be monitored according to any convenient method. Protein or mRNA can be monitored. Any technique known in the art for monitoring specific genes' expression can be used, including but not limited to ELISAs, SAGE, microarray hybridization, Western blots. Changes in expression of a single marker may be used as a criterion for significant effect as a potential pro-angiogenic, anti-angiogenic or anti-tumor agent. However, it also may be desirable to screen for test substances which are able to modulate the expression of at least 5, 10, 15, or 20 of the relevant markers, such as the tumor or normal endothelial markers. Inhibition of TEM protein activity can also be used as a drug screen. Human and mouse TEMS can be used for this purpose.

Test substances for screening can come from any source. They can be libraries of natural products, combinatorial chemical libraries, biological products made by recombinant libraries, etc. Nucleic acids and the corresponding encoded proteins of the markers described herein can be used therapeutically in a variety of modes. TEMs can be used to stimulate the growth of vasculature, such as for wound healing or to circumvent a blocked vessel. The nucleic acids and encoded proteins can be administered by any means known in the art. Such methods include, using liposomes, nanospheres, viral vectors, non-viral vectors comprising polycations, etc. Suitable viral vectors include adenovirus, retroviruses, and sindbis virus. Administration modes can be any known in the art, including parenteral, intravenous, intramuscular, intraperitoneal, topical, intranasal, intrarectal, intrabronchial, etc.

Specific biological antagonists of TEMs can also be used to therapeutic benefit. For example, antibodies, T cells specific for a TEM, antisense to a TEM, and ribozymes specific for a TEM can be used to restrict, inhibit, reduce, and/or diminish tumor or other abnormal or undesirable vasculature growth. Such antagonists can be administered as is known in the art for these classes of antagonists generally. Anti-angiogenic drugs and agents can be used to inhibit tumor growth, as well as to treat diabetic retinopathy, rheumatoid arthritis, psoriasis, polycystic kidney disease (PKD), and other diseases requiring angiogenesis for their pathologies.

The above disclosure generally describes the present invention. A more complete understanding can be obtained by reference to the following specific examples which are provided herein for purposes of illustration only, and are not intended to limit the scope of the invention.

### EXAMPLE 1

### Visualization of vasculature of colorectal cancers

The endothelium of human colorectal cancer was chosen to address the issues of tumor angiogenesis, based on the high incidence, relatively slow growth, and resistance to anti-neoplastic agents of these cancers. While certain less common tumor types, such as glioblastomas, are highly vascularized and are regarded as good targets for anti-angiogenic therapy, the importance of angiogenesis for the growth of human colorectal cancers and other common solid tumor types is less well documented.

We began by staining vessels in colorectal cancers using von Willebrand Factor (vWF) as a marker. In each of 6 colorectal tumors, this examination revealed a high density of vessels throughout the tumor parenchyma. Interestingly, these analyses also substantiated the importance of these vessels for tumor growth, as endothelium was often surrounded by a perivascular cuff of viable cells, with a ring of necrotic cells evident at the periphery. Although these preliminary studies suggested that colon tumors are angiogenesis-dependent, reliable markers that could distinguish vessels in colon cancers from the vessels in normal colon are currently lacking. One way to determine if such markers exist is by analyzing gene expression profiles in endothelium derived from normal and neoplastic tissue.

### EXAMPLE 2

### Purification of endothelial cells

Global systematic analysis of gene expression in tumor and normal endothelium has been hampered by at least three experimental obstacles. First, endothelium is enmeshed in a complex tissue consisting of vessel wall components, stromal cells, and neoplastic cells, requiring highly selective means of purifying ECs for analysis. Second, techniques for defining global gene expression profiles were not available until recently. And third, only a small fraction of the cells within a tumor are endothelial, mandating the development of methods that are suitable for the analysis of global expression profiles from relatively few cells.

To overcome the first obstacle, we initially attempted to purify ECs from dispersed human colorectal tissue using CD31, an endothelial marker commonly used for this purpose. This resulted in a substantial enrichment of ECs but also resulted in contamination of the preparations by hematopoietic cells, most likely due to expression of CD31 by macrophages. We therefore developed a new method for purifying ECs from human tissues using P1H12, a recently described marker for ECs. Unlike CD31, P1H12 was specifically expressed on the ECs of both colorectal tumors and normal colorectal mucosa. Moreover, immunofluorescence staining of normal and cancerous colon with a panel of known cell surface endothelial markers (e.g. VE-cadherin, CD31 and CD34) revealed that P1H12 was unique in that it stained all vessels including microvessels. In addition to selection with P1H12, it was necessary to optimize the detachment of ECs from their neighbors without destroying their cell surface proteins as well as to employ positive and negative affinity purifications using a cocktail of antibodies. The ECs purified from normal' colorectal mucosa and colorectal cancers were essentially free of epithelial and hematopoietic cells as judged by RT-PCR and subsequent gene expression analysis (see below).

### EXAMPLE 3

### Comparison of tumor and normal endothelial cell expression patterns

To overcome the remaining obstacles, a modification of the Serial Analysis of Gene Expression (SAGE) technique was used. SAGE associates individual mRNA transcripts with 14 base pair tags derived from a specific position near their 3' termini. The abundance of each tag provides a quantitative measure of the transcript level present within the mRNA population studied. SAGE is not dependent on pre-existing databases of expressed genes, and therefore provides an unbiased view of gene expression profiles. This feature is particularly important in the analysis of cells that constitute only a small fraction of the tissue under study, as transcripts from these cells are unlikely to be well represented in extant EST databases. We adapted the SAGE protocol so that it could be used on small numbers of purified ECs. A library of ~100,000 tags from the purified ECs of a colorectal cancer, and a similar library from the ECs of normal colonic mucosa from the same patient were generated. These ~193,000 tags corresponded to over 32,500 unique transcripts. Examination of the expression pattern of hematopoietic, epithelial and endothelial markers confirmed the purity of the preparations.

### EXAMPLE 4

### Tumor versus normal endothelium

We next attempted to identify transcripts that were differentially expressed in endothelium derived from normal or neoplastic tissues. Forty-seven tags encoding transmembrane proteins were identified that were expressed at 2-fold or higher levels in tumor vessels. Those transcripts expressed at higher levels in tumor endothelium are most likely to be useful in the future for diagnostic and therapeutic purposes.

### References and Notes

The disclosure of each reference cited is expressly incorporated herein.
1. J. Folkman, in Cancer Medicine J. Holland, Bast Jr, RC, Morton DL, Frei III, E, Kufe, DW, Weichselbaum, RR, Ed. (Williams & Wilkins, Baltimore, 1997) pp. 181.
2. R. S. Kerbel, Carcinogenesis 21, 505 (2000).
3. P. Wesseling, D. J. Ruiter, P. C. Burger, J Neurooncol 32, 253 (1997).
4. Q. G. Dong, et al., Arterioscler Thromb Vasc Biol 17, 1599 (1997).
5. P. W. Hewett, J. C. Murray, In Vitro Cell Dev Biol Anim 32, 462 (1996).
6. M. A. Hull, P. W. Hewett, J. L. Brough, C. J. Hawkey, Gastroenterology 111, 1230 (1996).
7. G. Haraldsen, et al., Gut 37, 225 (1995).
8. The original EC isolation protocol was the same as that shown in Fig. 2B except that dispersed cells were stained with anti-CD31 antibodies instead of anti-P1H12, and magnetic beads against CD64 and CD 14 were not included in the negative selection. After generating 120,000 SAGE tags from these two EC preparations, careful analysis of the SAGE data revealed that, in addition to endothelial-specific markers, several macrophage-specific markers were also present.
9. A. Solovey, et al., N Engl J Med 337, 1584 (1997).
10. V. E. Velculescu, L. Zhang, B. Vogelstein, K. W. Kinzler, Science 270 , 484-487 (1995).
11. In order to reduce the minimum amount of starting material required from ~50 million cells to ~50,000 cells (i.e. ~1000-fold less) we and others (38) have introduced several modifications to the original SAGE protocol. A detailed version of our modified "MicroSAGE" protocol is available from the authors upon request.
12. 96,694 and 96,588 SAGE tags were analyzed from normal and tumor derived ECs, respectively, and represented 50,298 unique tags. A conservative estimate of 32,703 unique transcripts was derived by considering only those tags observed more than once in the current data set or in the 134,000 transcripts previously identified in human transcriptomes (39).
13. To identify endothelial specific transcripts, we normalized the number of tags analyzed in each group to 100,000, and limited our analysis to transcripts that were expressed at levels at least 20-fold higher in ECs than in non-endothelial cell lines in culture and present at fewer than 5 copies per 100,000 transcripts in non-endothelial cell lines and the hematopoietic fraction (~57,000 tags)(41). Non-endothelial cell lines consisted of 1.8x106 tags derived from a total of 14 different cancer cell lines including colon, breast, lung, and pancreatic cancers, as well as one non-transformed keratinocyte cell line, two kidney epithelial cell lines, and normal monocytes. A complete list of PEMs is available at www.sagenet.org\angio\tablel.htm.
14. M. Tucci, et al., J Endocrinol 157, 13 (1998).
15. T. Oono, et al., J Invest Dermatol 100, 329 (1993).
16. K. Motamed, Int J Biochem Cell Biol 31, 1363 (1999).
17. N. Bardin, et al., Tissue Antigens 48, 531 (1996).
18. D. M. Bradham, A. Igarashi, R. L. Potter, G. R. Grotendorst, J Cell Biol 114, 1285 (1991).
19. K. Akaogi, et al., Proc Natl Acad Sci U S A 93, 8384 (1996).
20. Y. Muragaki, et al., Proc Natl Acad Sci U S A 92, 8763 (1995).
21. M. L. Iruela-Arispe, C. A. Diglio, E. H. Sage, Arterioscler Thromb 11 , 805 (1991).
22. J. P. Girard, T. A. Springer, Immunity 2,113 (1995).
23. E. A. Jaffe, et al., J Immunol 143, 3961 (1989).
24. J. P. Girard, et al., Am J Pathol 155, 2043 (1999).
25. H. Ohtani, N. Sasano, J Electron Microsc 36, 204 (1987).
26. For non-radioactive in situ hybridization, digoxigenin (DIG)-labelled sense and anti-sense riboprobes were generated through PCR by amplifying 500-600 bp products and incorporating a T7 promoter into the anti-sense primer. In vitro transcription was performed using DIG RNA labelling reagents and T7 RNA polymerase (Roche, Indianapolis, IN). Frozen tissue sections were fixed with 4 % paraformaldehyde, permeabilized with pepsin, and incubated with 200 ng/ml of riboprobe overnight at 55°C. For signal amplification, a horseradish peroxidase (HRP) rabbit anti-DIG antibody (DAKO, Carpinteria, CA) was used to catalyse the deposition of Biotin-Tyramide (from GenPoint kit, DAKO). Further amplification was achieved by adding HRP rabbit anti-biotin (DAKO), biotin-tyramide, and then alkaline-phosphatase (AP) rabbit anti-biotin (DAKO). Signal was detected using the AP substrate Fast Red TR/Napthol AS-MX (Sigma, St. Louis, MO), and cells were counterstained with hematoxylin unless otherwise indicated. A detailed protocol including the list of primers used to generate the probes can be obtained from the authors upon request.
27. Transcript copies per cell were calculated assuming an average cell contains 300,000 transcripts.
28. R. S. Warren, H. Yuan, M. R. Matli, N. A. Gillett, N. Ferrara, J Clin Invest 95, 1789 (1995).
29. Y. Takahashi, Y. Kitadai, C. D. Bucana, K. R. Cleary, L. M. Ellis, Cancer Res 55, 3964 (1995).
30. L. F. Brown, et al., Cancer Res 53, 4727 (1993).
31. Endothelial-specific transcripts were defined as those expressed at levels at least 5-fold higher in ECs in vivo than in non-endothelial cell lines in culture (13), and present at no more than 5 copies per 100,000 transcripts in non-endothelial cell lines and the hematopoietic cell fraction (41). Transcripts showing statistically different levels of expression (P <0.05) were then identified using Monte Carlo analysis as previously described (40). Transcripts preferentially expressed in normal endothelium were then defined as those expressed at levels at least 10-fold higher in normal endothelium than in tumor endothelium. Conversely, tumor endothelial transcripts were at least 10-fold higher in tumor versus normal endothelium. See www.sagenet.org\angio\table2.htm and www.sagenet.org\angio\table3.htm for a complete list of differentially expressed genes.
32. M. Iurlaro, et al., Eur J Clin Invest 29, 793 (1999).
33. W. S. Lee, et al., Circ Res 82, 845 (1998).
34. J. Niquet, A. Represa, Brain Res Dev Brain Res 95, 227 (1996).
35. L. Fouser, L. Iruela-Arispe, P. Bornstein, E. H. Sage, J Biol Chem 266 , 18345 (1991).
36. M. L. Iruela-Arispe, P. Hasselaar, H. Sage, Lab Invest 64, 174 (1991).
37. H. F. Dvorak, N Engl J Med 315, 1650 (1986).
38. B. Virlon, et al., Proc Natl Acad Sci USA 96, 15286 (1999).
39. V. E. Velculescu, et al., Nat Genet 23, 387 (1999).
40. L. Zhang, et al., Science 276, 1268 (1997).
41. Human colon tissues were obtained within ½ hour after surgical removal from patients. Sheets of epithelial cells were peeled away from normal tissues with a glass slide following treatment with 5 mM DDT, then 10 mM EDTA, leaving the lamina propria intact. After a 2h incubation in collagenase at 37 oC, cells were filtered sequentially through 400 um, 100 um, 50 um and 25 um mesh, and spun through a 30 % pre-formed Percoll gradient to pellet RBCs. Epithelial cells (Epithelial Fraction), which were found to non-specifically bind magnetic beads, were removed using Dynabeads coupled to BerEP4 (Dynal, Lake Success, NY). Subsequently, macrophages and other leukocytes (Hematopoietic Fraction) were removed using a cocktail of beads coupled to anti-CD45, anti-CD14 and anti-CD64 (Dynal). The remaining cells were stained with P1H12 antibody, purified with anti-mouse IgG-coupled magnetic beads, and lysed in mRNA lysis buffer. A detailed protocol can be obtained from the authors upon request.
42. H. Sheikh, H. Yarwood, A. Ashworth, C. M. Isacke, J Cell Sci 113, 1021-32 (2000).

**Table 1. Membrane-associated tumor endothelial markers**

| Unigene ID | Function | OMIMID | Signal Seq | Protein | TM Location | Orientation | SEQ ID NO |
|---|---|---|---|---|---|---|---|
| Hs.102308 | potassium inwardly-rectifying channel, subfamily J, member 8 | 600935 | no | NP_004973 | 73-95,156-178 | IN | |
| Hs.109225 | Vascular cell adhesion molecule 1 | 192225 | yes | NP_001069 | 699-721 | Unsure | |
| Hs.110024 | NADH:ubiquinone oxidoreductase MLRQ subunit homolog | | yes | NP_064527 | 20-42 | Unsure | |
| Hs.125036 | TEM17 | 606826 | yes | NP 065138 | 425-447 | OUT | |
| Hs.125359 | TEM13, Thy-1 cell surface antigen | 188230 | yes | NP_006279 | 140-161 | Unsure | |
| His.13662 | hypothetical protein MGC5508 | | yes | NP_076997 | 84-106,130-152,159-176,186-205 | Unsure | |
| Hs.1501 | syndecan 2 (heparan sulfate proteoglycan 1, cell surface-associated, fibroglycan) | 142460 | yes | AAA52701 | 147-169 | Unsure | |
| Hs.150540 | hypothetical protein BC002942 | | yes | NP_149977 | 367-389,314-336,79-101,256-278,108-130,401-423,639-661,131-152,13-35 226-248 | Unsure | |
| Hs.155071 | TEM44, hypothetical protein FLJ11190 | | no | NP_060824 | 121-143,177-199 | Unsure | |
| Hs.16187 | uncharacterized hematopoietic stem/progenitor cells protein MDS032 | | no | NP_060937 | 232-254 | OUT | |
| Hs.166994 | FAT tumor suppressor homolog 1 (Drosophila) | 600976 | yes | NP_005236 | 4181-4203 | Unsure | |
| Hs.17170 | G protein-coupled receptor 4 | 600551 | no | NP_005273 | 55-77,92-113,20-42,225-244,183-205 | OUT | |
| Hs.17270 | TEM9 | 606823 | yes | NP_116166 | 921-943,764-786,1041-1060,878-900,799-821_1012-1034 | Unsure | |
| Hs.177486 | amyloid beta (A4) precursor protein (protease nexin-II, Alzheimer disease) | 104760 | yes | NP_000475 | 701-723 | Unsure | |
| Hs.180338 | tumor necrosis factor receptor superfamily, formerly member 12, now member 25 (translocating chain-association membrane protein) | 603366 | yes | NP_683869 | 200-222 | IN | 9, 10 |
| Hs.181244 | histocompatibility complex, class I. A | 142800 | yes | NP_002107 | 305-327 | OUT | |
| Hs.185973 | degenerative spermatocyte homolog, lipid desaturase (Drosophila) | | yes | NP_003667 | 43-61,160-177 | Unsure | |
| Hs.195727 | TEM1, endosialin | 606064 | yes | NP 065137 | 686-708 | Unsure | |
| Hs.198265 | matrix metalloproteinase 25 | | yes | NP_071913 | 541-562 | Unsure | |
| Hs.20166 | prostate stem cell antigen | 602470 | yes | NP_005663 | 100-122 | Unsure | |
| Hs.211579 | melanoma cell adhesion molecule | 155735 | yes | NP_006491 | 560-582 | OUT | |
| Hs.23016 | G protein-coupled receptor | | yes | | 47-69,297-319,82-104,214-236,119-140,160-182, 255-277 | OUT | 3, 4 |
| Hs.231119 | protocadherin beta 9 | 606335 | yes | NP_061992 | 689-711,13-35 | IN | |
| Hs.2399 | matrix metalloproteinase 14 (membrane-inserted) | 600754 | yes | NP_004986 | 540-562 | Unsure | |
| Hs.24220 | Scotin | 607290 | ves | NP_057563 | 110-132 | OUT | |
| Hs.24395 | chemokine (C-X-C motif) ligand 14 | 604186 | no | NP_004878 | 31-Oct | OUT | |
| Hs.251385 | murine retrovirus integration site 1 homolog | 604673 | no | NP_569056 | 830-852 | Unsure | |
| Hs.256297 | integrin, alpha 11 | 604789 | yes | NP_036343 | 1143-1165 | OUT | |
| Hs.265827 | interferon, alpha-inducible protein (clone IFI-6-16) | 147572 | yes | NP_075011 | 5-24,44-66 | IN | |
| Hs.274127 | CLST 11240 protein | | no | NP_057522 | 62-84,30-47 | IN | |
| Hs.274368 | TEM42, MSTP032 rev str: | | no | NP_079502 | 47-69 | OUT | 1, 2 |
| Hs.278568 | H factor (complement)-like 1 | 134371 | yes | NP_002104 | 23-Jan | Unsure | |
| Hs.27935 | tweety homolog 2 (Drosophila) | | yes | NP_116035 | 242-264,89-111,390-412,215-237,47-69 | IN | |
| Hs.279746 | transient receptor potential cation channel, subfamily V, member 2 | 606676 | no | NP_057197 | 535-557,391-413,492-514,433-455,622-644, 460-482 | Unsure | |
| Hs.283558 | Hypothetical protein PRO185 | | no | NP_060979 | 246-268 | IN | |
| Hs.285814 | sprouty homolog 4 (Drosophila) | | no | AAK00653 | 236-258 | OUT | |
| Hs.291904 | accessory protein BAP31 | 300398 | yes | NP_005736 | 44-63, 102-121 | IN | |
| Hs.295726 | integrin, alpha V (vitronectin receptor, alpha polypeptide, antigen CD51) | 193210 | yes | NP_002201 | 994-1016 | OUT | |
| Hs.296310 | gap junction protein, alpha 4, 37kDa (connexin 37) | 121012 | no | NP_002051 | 207-229,20-39,76-98 | IN | |
| Hs.29665 | calsyntenin 1 | | yes | NP_055759 | 860-882 | Unsure | |
| Hs.298476 | solute carrier family 26, member 6 | | no | NP_599025 | 380-402,187-209,115-137,475-506,417-436,264-283,346-368,141-163,295-314,443 460 | OUT | |
| Hs.29882 | family with sequence similarity 3, member C | | yes | NP_055703 | 29-Jul | IN | |
| Hs.300697 | immunoglobulin heavy constant gamma 3 (G3m | 147120 | yes | | 547-569 | OUT | |
| Hs.31720 | marker) Hephaestin | 300167 | yes | NP_620074 | 1108-1130 | OUT | |
| Hs.322456 | Hypothetical protein DKFZp761D0211 | | no | NP_114428 | 49-71 | IN | |
| Hs.323769 | cisplatin resistance related protein CRR9p | | yes | NP_110409 | 15-36,401-423,285-307,431-453 ,345-362,318-340 | IN | |
| Hs.324844 | Hypothetical protein IMAGE3455200 | | yes | NP_076869 | 75-97,101-123.116-138 | Unsure | |
| Hs.34665 | Homo sapiens mRNA full length insert cDNA clone EUROIMAGE88179 1 | | no | | 456-478 | OUT | |
| Hs.381200 | Hypothetical protein MGC15523 | | yes | NP_612637 | 378-397,83-105,120-142,230-252,323-340,149-171,344-366,272-294,36-58 | IN | |
| Hs.393 | Prostaglandin I2 (prostacyclin) receptor (IP) | 600022 | no | NP_000951 | 188-210,49-71,93-115,136-158,238-260,15-37 | OUT | |
| Hs.43910 | CD164 antigen, sialomucin | 603356 | yes | NP_006007 | 164-186 | Unsure | |
| Hs.6459 | Putative G-protein coupled receptor GPCR41 | | yes | NP_078807 | 196-218,46-68,369-391,81-103,113-135,404-426,147-169,325-347,337-359,9-31,276, 298 | IN | |
| Hs.7158 | DKFZP566H073 protein | | yes | NP_056343 | 172-194 | Unsure | |
| Hs.746155 | platelet-derived growth factor receptor, alpha polypeptide | 173490 | yes | NP_006197 | 527-549,7-29 | IN | |
| Hs.74823 | NADH dehydrogenase (ubiquinone) 1 alpha subcomplex, 1,75kDa | 300078 | yes | NP_004532 | 27-May | OUT | |
| Hs.75564 | CD151 antigen | 602243 | yes | | 57-79,92-114,222-244 | IN | |
| Hs.76144 | platelet-derived growth factor receptor, beta polypeptide | 173410 | yes | NP_002600 | 534-556 | Unsure | |
| Hs.77665 | KIAA0102 gene product | product | no | NP_055567 | 80-102,112-134 | IN | |
| Hs.77873 | B7 homolog 3 | 605715 | yes | | 466-488 | IN | |
| Hs.7835 | TEM22, endocytic receptor (mannose receptor, C type 2); involved in cell-cell communication, cell adhesion | | yes | NP_006030 | 1412-1434 | OUT | |
| Hs.79410 | solute carrier family 4, anion exchanger, member 2 (erythrocyte membrane protein band 3-like 1) | 109280 | no | NP_003031 | 794-816,1031-1053,901-918,709-731,988-1010,752-774,818-840,931-950,1114-1136,1175-1197,1188-1310 1101-1103 | OUT | |
| Hs.82002 | endothelin receptor type B | 131244 | yes | NP_000106 | 367-389,104-126,217-239,138-160,325-347,175-197,275-297 | Unsure | 5, 6 |
| Hs.82890 | defender against cell death 1 | 600243 | yes | NP_001335 | 29-51,56-78,93-112 | OUT | |
| Hs.83883 | transmembrane, prostate androgen induced RNA | 606564 | yes | NP_064567 | 41-63 | OUT | |
| Hs.8546 | Notch homolog 3 (Drosophila) | 600276 | yes | NP_000426 | 1641-1663,1496-1518.20-42 | Unsure | 7,8 |
| Hs.890 | lymphotoxin beta (TNF superfamily, member 3) | 600978 | yes | NP_002332 | 21-43 | IN | |
| Hs.8966 | TEM19 var1 (long); cell-surface protein, domain homology with leukointegrin (integrin alpha-D); ATR | 606410 | yes | NP_115584 | 321-343 | IN | |
| Hs.9004 | chondroitin sulfate proteoglycan (melanoma-associated) | 601172 | yes | NP_001888 | 2224-2246 | Unsure | |
| Hs.93765 | lipoma HMGIC fusion partner | 606710 | yes | NP_005771 | 87-109,121-143,12-34,166-188 | Unsure | |
| Hs.95744 | hypothetical protein similar to ankyrin repeat-containing priotein AKR1 | | no | NP_061901 | 472-494,289-311,318-340,347-369,374-395,505-528 | OUT | |
| Hs.17144 | short-chain dehydrogenase/red uctase 1 SDR1 | | yes | NP_004744 | | | |
| Hs.32978 | proprotein convertase subtilisin/kexin type 7 PCSK7 | 604872 | yes | NP_004707 | | | |
| Hs,289770 | Homo sapiens mRNA; cDNA DKF2p686D0720 | | no | | | | |
| | | | | | | | |
| | | | | | | | |
| | | | | | | | |
| | | | | | | | |
| | | | | | | | |
| | | | | | | | |
| Hs.418 | fibroblast activation protein, alpha FAP | 600403 | yes | NP_004451 | | | |
| Hs.211579 | melanoma cell adhesion molecule mCAM | 155735 | yes | NP_006491 | | | |
| Hs.9383 | cystein-rich with EFG-like domains 1 CRELD1 | 607170 | yes | NP_056328 | | | |

## Claims

1. A method for detecting tumor endothelium in a patient, comprising:
contacting cDNA or mRNA obtained from a population of colorectal endothelial cells obtained from the patient with one or more nucleic acid hybridization probes which are complementary to a cDNA or mRNA for a gene encoding an extracellular domain of the FAP fibroblast activation protein, alpha; and
detecting cDNA or mRNA which have specifically hybridized to said nucleic acid hybridization probes,
wherein the detection of higher than normal levels of the cDNA or mRNA indicates tumor endothelium.

## Patentansprüche

1. Verfahren zum Nachweis von Tumorendothel bei einem Patienten, umfassend:
In Kontakt bringen von cDNA oder mRNA, die aus einer von dem Patienten erhaltenen Population von kolorektalen Endothelzellen erhalten wurde, mit ein oder mehreren Sonden zur Nukleinsäurehybridisierung, die komplementär zu einer cDNA oder mRNA für ein Gen sind, das für eine extrazelluläre Domäne des FAP Fibroblasten-Aktivierungsproteins alpha kodiert; und
Nachweis von cDNA oder mRNA, die spezifisch an die Sonden zur Nukleinsäurehybridisierung hybridisiert hat,
wobei der Nachweis höherer als normaler Mengen der cDNA oder mRNA Tumorendothel anzeigt.

## Revendications

1. Procédé de détection d'un endothélium tumoral chez un patient, comprenant :
le contact d'un ADNc ou d'un ARNm provenant d'une population de cellules endothéliales colorectales provenant du patient avec une ou plusieurs sondes d'hybridation d'acide nucléique qui sont complémentaires à un ADNc ou à un ARNm pour un gène codant un domaine extracellulaire de la protéine alpha d'activation des fibroblastes FAP ; et
la détection des ADNc ou des ARNm qui se sont hybridés spécifiquement auxdites sondes d'hybridation d'acide nucléique,
dans lequel la détection de taux plus élevés que les taux normaux de l'ADNc ou de l'ARNm indique un endothélium tumoral.


### SEQUENCE LISTING

<110> St. Croix, Brad
   Kinzler, Kenneth W.
   Vogelstein, Bert
<120> MEMBRANE ASSOCIATED TUMOR ENDOTHELIUM MARKERS
<130> 001107.00358
<150> 60/390,187
   <151> 2002-06-21
<160> 10
<170> FastSEQ for Windows version 4.0
<210> 1
   <211> 1909
   <212> DNA
   <213> Homo sapiens
<400> 1
   gacacctttt aaaatgcaga actaactgag gcatttcagt aactttgctt tcaaatcaat      60
   aaagtcaaat gtatggaaac attttgtgcc ctactctcca taccctgtgt actcaaattc     120
   tctactgtat gaattatgct ttaagtagaa ttcagtgcca aggagaactt ggtgaaataa     180
   attattttaa tttttttttt atcctttaca aagccatgga ttttatttgg ttgatgtgtg     240
   ctctgtacac aagccatttc aataggatgg agctgttaat tattttccaa agagtaatag     300
   acatgcaaaa gtttcaataa aaactgggcc attaacaaat aaattaataa actaataagc     360
   attcccttct aggtttttgc caaactgcct atccaataac aaatttgaga atcgttgaaa     420
   aagctagtta tatttcagag aaatgatttt cattattgaa actgttctcc ctagcaggcc     480
   attttccctt tttcctggga gtttagcaag tttaggagag aatagtcatg aaaagaaagg     540
   gaagaaaggg gagaagggaa gaggttaaaa agtaagtgct cagacctatg aacgtaatcc     600
   ctttgctaga aatatttaag agcagctcag cttggttgaa actgagtttt gtcatcttcc     660
   atatttgcag gaaggtattt tctgacttgc aatgcagcta gatgtaaaat tttattttat     720
   catcctagaa agccttgact agaaaaatga ataaatattg agggtttcct gtccatatct     780
   ggcttgcatg tgccagaaag cagagaatag aaaatgtaat ctccaacatc caagcatcga     840
   aacccaaggg gtaggcaatt ctatgtaggt tttggacatg aagtttggtg catcttggtt     900
   tatgctggct caactgctat taaacctctc tggcttatag tctcttcatt ctattagaca     960
   agcacgtatc gaacacttgc ttcgcacaag gctctttagt taacaattta gcagctactg    1020
   tttgtgttaa acacactttt caccaaatag gttctgaggc aaacgagagc aatgactatt    1080
   taaagaaagg ctttcccagc atcacttaca catcccaaaa ctaaaaagat caactcttcc    1140
   aactgagaaa agactcctgg ctttgaatgg aaacttacag cagagagtca caggccacgg    1200
   caacaacaac gacaacaaca aacatttgga atattattct caactcacgt tttaataata    1260
   catcttaatt atttttctag tagagaaact acaaatcagc ctcttcaaca tttatataca    1320
   gtttaataag cctcttgcaa gttacttgtt ctctcacctg aggtattttt ttcctcccca    1380
   ccttgcccct gttcctccct tcctcttctc cctttgcaag aggaaatatt taacatattt    1440
   gggtccaact tcaataatgt aataattaat acattaaaag catttaactt cctttctaga    1500
   aaaatgcaca ggctaaggca tagacaaaac aaagagaaat gctgagaaat ttgccactgg    1560
   agacaagcaa tctgaataaa tatttgccaa aagttctttt tatgtcatat agtgtcagga    1620
   tttgaaggag ctattttttt taatgttgca actagcaact catcttcgga agacacagcc    1680
   aggagaatga agtagaagtg aaaggtttat aaatccattt gtaagcattt atcccatata    1740
   ttttaaattc aagaaaaatt gtgtttatct ttagaatttt gtattcaata ctttatgtac    1800
   tatgtgactc atgcttctgg ataaataaag caccaaatat gtatctgtaa ccacaatcac    1860
   acatattata ttaaatatat atctatataa caaaaaaaaa aaaaaaaaa                1909
<210> 2
   <211> 83
   <212> PRT
   <213> Homo sapiens
<400> 2
   Met Tyr Gly Asn Ile Leu Cys Pro Thr Leu His Thr Leu Cys Thr Gln
    1               5                  10                  15
   Ile Leu Tyr Cys Met Asn Tyr Ala Leu Ser Arg Ile Gln Cys Gln Gly
               20                  25                  30
   Glu Leu Gly Glu Ile Asn Tyr Phe Asn Phe Phe Phe Ile Leu Tyr Lys
           35                  40                  45
   Ala Met Asp Phe Ile Trp Leu Met Cys Ala Leu Tyr Thr Ser His Phe
       50                  55                  60
   Asn Arg Met Glu Leu Leu Ile Ile Phe Gln Arg Val Ile Asp Met Gln
   65                  70                  75                  80
   Lys Phe Gln
<210> 3
   <211> 2064
   <212> DNA
   <213> Homo sapiens
<400> 3
   tgcaagtctg cagccagcag agctcacagt tgttgcaaag tgctcagcac taagggagcc      60
   agcgcacagc acagccagga aggcgagcga gcccagccag cccagccagc ccagccagcc     120
   cggaggtcat ttgattgccc gcctcagaac gatggatctg catctcttcg actactcaga     180
   gccagggaac ttctcggaca tcagctggcc atgcaacagc agcgactgca tcgtggtgga     240
   cacggtgatg tgtcccaaca tgcccaacaa aagcgtcctg ctctacacgc tctccttcat     300
   ttacattttc atcttcgtca tcggcatgat tgccaactcc gtggtggtct gggtgaatat     360
   ccaggccaag accacaggct atgacacgca ctgctacatc ttgaacctgg ccattgccga     420
   cctgtgggtt gtcctcacca tcccagtctg ggtggtcagt ctcgtgcagc acaaccagtg     480
   gcccatgggc gagctcacgt gcaaagtcac acacctcatc ttctccatca acctcttcgg     540
   cagcattttc ttcctcacgt gcatgagcgt ggaccgctac ctctccatca cctacttcac     600
   caacaccccc agcagcagga agaagatggt acgccgtgtc gtctgcatcc tggtgtggct     660
   gctggccttc tgcgtgtctc tgcctgacac ctactacctg aagaccgtca cgtctgcgtc     720
   caacaatgag acctactgcc ggtccttcta ccccgagcac agcatcaagg agtggctgat     780
   cggcatggag ctggtctccg ttgtcttggg ctttgccgtt cccttctcca ttatcgctgt     840
   cttctacttc ctgctggcca gagccatctc ggcgtccagt gaccaggaga agcacagcag     900
   ccggaagatc atcttctcct acgtggtggt cttccttgtc tgctggctgc cctaccacgt     960
   ggcggtgctg ctggacatct tctccatcct gcactacatc cctttcacct gccggctgga    1020
   gcacgccctc ttcacggccc tgcatgtcac acagtgcctg tcgctggtgc actgctgcgt    1080
   caaccctgtc ctctacagct tcatcaatcg caactacagg tacgagctga tgaaggcctt    1140
   catcttcaag tactcggcca aaacagggct caccaagctc atcgatgcct ccagagtctc    1200
   agagacggag tactctgcct tggagcagag caccaaatga tctgccctgg agaggctctg    1260
   ggacgggttt acttgttttt gaacagggtg atgggcccta tggttttcta gagcaaagca    1320
   aagtagcttc gggtcttgat gcttgagtag agtgaagagg ggagcacgtg ccccctgcat    1380
   ccattctctc tttctcttga tgacgcagct gtcatttggc tgtgcgtgct gacagttttg    1440
   caacaggcag agctgtgtcg cacagcagtg ctgtgcgtca gagccagctg aggacaggct    1500
   tgcctggact tctgtaagat aggattttct gtgtttcctg aattttttat atggtgattt    1560
   gtatttaaat tttaagactt tattttctca ctattggtgt accttataaa tgtatttgaa    1620
   agttaaatat attttaaata ttgtttggga ggcatagtgc tgacatatat tcagagtgtt    1680
   gtagttttaa ggttagcgtg acttcagttt tgactaagga tgacactaat tgttagctgt    1740
   tttgaaatta tatatatata aatatatata aatatataaa tatatgccag tcttggctga    1800
   aatgttttat ttaccatagt tttatatctg tgtggtgttt tgtaccggca cgggatatgg    1860
   aacgaaaact gctttgtaat gcagtttgtg acattaatag tattgtaaag ttacatttta    1920
   aaataaacaa aaaactgttc tggactgcaa atctgcacac acaacgaaca gttgcatttc    1980
   agagagttct ctcaatttgt aagttatttt tttttaataa agatttttgt ttccaaaaaa    2040
   aaaaaaaaaa aaaaaaaaaa aaaa                                           2064
<210> 4
   <211> 362
   <212> PRT
   <213> Homo sapiens
<400> 4
   Met Asp Leu His Leu Phe Asp Tyr Ser Glu Pro Gly Asn Phe Ser Asp
   1               5                   10                  15
   Ile Ser Trp Pro Cys Asn Ser Ser Asp Cys Ile Val Val Asp Thr Val
               20                  25                  30
   Met Cys Pro Asn Met Pro Asn Lys Ser Val Leu Leu Tyr Thr Leu Ser
           35                  40                  45
   Phe Ile Tyr Ile Phe Ile Phe Val Ile Gly Met Ile Ala Asn Ser Val
       50                  55                  60
   Val Val Trp Val Asn Ile Gln Ala Lys Thr Thr Gly Tyr Asp Thr His
   65                  70                  75                  80
   Cys Tyr Ile Leu Asn Leu Ala Ile Ala Asp Leu Trp Val Val Leu Thr
                   85                  90                  95
   Ile Pro Val Trp Val Val Ser Leu Val Gln His Asn Gln Trp Pro Met
               100                 105                 110
   Gly Glu Leu Thr Cys Lys Val Thr His Leu Ile Phe Ser Ile Asn Leu
           115                 120                 125
   Phe Gly Ser Ile Phe Phe Leu Thr Cys Met Ser Val Asp Arg Tyr Leu
       130                 135                 140
   Ser Ile Thr Tyr Phe Thr Asn Thr Pro Ser Ser Arg Lys Lys Met Val
   145                 150                 155                 160
   Arg Arg Val Val Cys Ile Leu Val Trp Leu Leu Ala Phe Cys Val Ser
                   165                 170                 175
   Leu Pro Asp Thr Tyr Tyr Leu Lys Thr Val Thr Ser Ala Ser Asn Asn
               180                 185                 190
   Glu Thr Tyr Cys Arg Ser Phe Tyr Pro Glu His Ser Ile Lys Glu Trp
           195                 200                 205
   Leu Ile Gly Met Glu Leu Val Ser Val Val Leu Gly Phe Ala Val Pro
       210                 215                 220
   Phe Ser Ile Ile Ala Val Phe Tyr Phe Leu Leu Ala Arg Ala Ile Ser
   225                 230                 235                 240
   Ala Ser Ser Asp Gln Glu Lys His Ser Ser Arg Lys Ile Ile Phe Ser
                   245                 250                 255
   Tyr Val Val Val Phe Leu Val Cys Trp Leu Pro Tyr His Val Ala Val
               260                 265                 270
   Leu Leu Asp Ile Phe Ser Ile Leu His Tyr Ile Pro Phe Thr Cys Arg
           275                 280                 285
   Leu Glu His Ala Leu Phe Thr Ala Leu His Val Thr Gln Cys Leu Ser
       290                 295                 300
   Leu Val His Cys Cys Val Asn Pro Val Leu Tyr Ser Phe Ile Asn Arg
   305                 310                 315                 320
   Asn Tyr Arg Tyr Glu Leu Met Lys Ala Phe Ile Phe Lys Tyr Ser Ala
                   325                 330                 335
   Lys Thr Gly Leu Thr Lys Leu Ile Asp Ala Ser Arg Val Ser Glu Thr
               340                 345                 350
   Glu Tyr Ser Ala Leu Glu Gln Ser Thr Lys
           355                 360
<210> 5
   <211> 4286
   <212> DNA
   <213> Homo sapiens
<400> 5
   gagacattcc ggtgggggac tctggccagc ccgagcaacg tggatcctga gagcactccc      60
   aggtaggcat ttgccccggt gggacgcctt gccagagcag tgtgtggcag gcccccgtgg     120
   aggatcaaca cagtggctga acactgggaa ggaactggta cttggagtct ggacatctga     180
   aacttggctc tgaaactgcg cagcggccac cggacgcctt ctggagcagg tagcagcatg     240
   cagccgcctc caagtctgtg cggacgcgcc ctggttgcgc tggttcttgc ctgcggcctg     300
   tcgcggatct ggggagagga gagaggcttc ccgcctgaca gggccactcc gcttttgcaa     360
   accgcagaga taatgacgcc acccactaag accttatggc ccaagggttc caacgccagt     420
   ctggcgcggt cgttggcacc tgcggaggtg cctaaaggag acaggacggc aggatctccg     480
   ccacgcacca tctcccctcc cccgtgccaa ggacccatcg agatcaagga gactttcaaa     540
   tacatcaaca cggttgtgtc ctgccttgtg ttcgtgctgg ggatcatcgg gaactccaca     600
   cttctgagaa ttatctacaa gaacaagtgc atgcgaaacg gtcccaatat cttgatcgcc     660
   agcttggctc tgggagacct gctgcacatc gtcattgaca tccctatcaa tgtctacaag     720
   ctgctggcag aggactggcc atttggagct gagatgtgta agctggtgcc tttcatacag     780
   aaagcctccg tgggaatcac tgtgctgagt ctatgtgctc tgagtattga cagatatcga     840
   gctgttgctt cttggagtag aattaaagga attggggttc caaaatggac agcagtagaa     900
   attgttttga tttgggtggt ctctgtggtt ctggctgtcc ctgaagccat aggttttgat     960
   ataattacga tggactacaa aggaagttat ctgcgaatct gcttgcttca tcccgttcag    1020
   aagacagctt tcatgcagtt ttacaagaca gcaaaagatt ggtggctgtt cagtttctat    1080
   ttctgcttgc cattggccat cactgcattt ttttatacac taatgacctg tgaaatgttg    1140
   agaaagaaaa gtggcatgca gattgcttta aatgatcacc taaagcagag acgggaagtg    1200
   gccaaaaccg tcttttgcct ggtccttgtc tttgccctct gctggcttcc ccttcacctc    1260
   agcaggattc tgaagctcac tctttataat cagaatgatc ccaatagatg tgaacttttg    1320
   agctttctgt tggtattgga ctatattggt atcaacatgg cttcactgaa ttcctgcatt    1380
   aacccaattg ctctgtattt ggtgagcaaa agattcaaaa actgctttaa gtcatgctta    1440
   tgctgctggt gccagtcatt tgaagaaaaa cagtccttgg aggaaaagca gtcgtgctta    1500
   aagttcaaag ctaatgatca cggatatgac aacttccgtt ccagtaataa atacagctca    1560
   tcttgaaaga agaactattc actgtatttc attttcttta tattggaccg aagtcattaa    1620
   aacaaaatga aacatttgcc aaaacaaaac aaaaaactat gtatttgcac agcacactat    1680
   taaaatatta agtgtaatta ttttaacact cacagctaca tatgacattt tatgagctgt    1740
   ttacggcatg gaaagaaaat cagtgggaat taagaaagcc tcgtcgtgaa agcacttaat     1800
   tttttacagt tagcacttca acatagctct taacaacttc caggatattc acacaacact     1860
   taggcttaaa aatgagctca ctcagaattt ctattctttc taaaaagaga tttattttta     1920
   aatcaatggg actctgatat aaaggaagaa taagtcactg taaaacagaa cttttaaatg     1980
   aagcttaaat tactcaattt aaaattttaa aatcctttaa aacaactttt caattaatat     2040
   tatcacacta ttatcagatt gtaattagat gcaaatgaga gagcagttta gttgttgcat     2100
   ttttcggaca ctggaaacat ttaaatgatc aggagggagt aacagaaaga gcaaggctgt     2160
   ttttgaaaat cattacactt tcactagaag cccaaacctc agcattctgc aatatgtaac     2220
   caacatgtca caaacaagca gcatgtaaca gactggcaca tgtgccagct gaatttaaaa     2280
   tataatactt ttaaaaagaa aattattaca tcctttacat tcagttaaga tcaaacctca     2340
   caaagagaaa tagaatgttt gaaaggctat cccaaaagac ttttttgaat ctgtcattca    2400
   cataccctgt gaagacaata ctatctacaa ttttttcagg attattaaaa tcttcttttt    2460
   tcactatcgt agcttaaact ctgtttggtt ttgtcatctg taaatactta cctacataca     2520
   ctgcatgtag atgattaaat gagggcaggc cctgtgctca tagctttacg atggagagat     2580
   gccagtgacc tcataataaa gactgtgaac tgcctggtgc agtgtccaca tgacaaaggg     2640
   gcaggtagca ccctctctca cccatgctgt ggttaaaatg gtttctagca tatgtataat     2700
   gctatagtta aaatactatt tttcaaaatc atacagatta gtacatttaa cagctacctg     2760
   taaagcttat tactaatttt tgtattattt ttgtaaatag ccaatagaaa agtttgcttg     2820
   acatggtgct tttctttcat ctagaggcaa aactgctttt tgagaccgta agaacctctt     2880
   agctttgtgc gttcctgcct aatttttata tcttctaagc aaagtgcctt aggatagctt     2940
   gggatgagat gtgtgtgaaa gtatgtacaa gagaaaacgg aagagagagg aaatgaggtg     3000
   gggttggagg aaacccatgg ggacagattc ccattcttag cctaacgttc gtcattgcct     3060
   cgtcacatca atgcaaaagg tcctgatttt gttccagcaa aacacagtgc aatgttctca     3120
   gagtgacttt cgaaataaat tgggcccaag agctttaact cggtcttaaa atatgcccaa     3180
   atttttactt tgtttttctt ttaataggct gggccacatg ttggaaataa gctagtaatg     3240
   ttgttttctg tcaatattga atgtgatggt acagtaaacc aaaacccaac aatgtggcca     3300
   gaaagaaaga gcaataataa ttaattcaca caccatatgg attctattta taaatcaccc     3360
   acaaacttgt tctttaattt catcccaatc actttttcag aggcctgtta tcatagaagt     3420
   cattttagac tctcaatttt aaattaattt tgaatcacta atattttcac agtttattaa     3480
   tatatttaat ttctatttaa attttagatt atttttatta ccatgtactg aatttttaca     3540
   tcctgatacc ctttccttct ccatgtcagt atcatgttct ctaattatct tgccaaattt     3600
   tgaaactaca cacaaaaagc atacttgcat tatttataat aaaattgcat tcagtggctt     3660
   tttaaaaaaa atgtttgatt caaaacttta acatactgat aagtaagaaa caattataat     3720
   ttctttacat actcaaaacc aagatagaaa aaggtgctat cgttcaactt caaaacatgt     3780
   ttcctagtat taaggacttt aatatagcaa cagacaaaat tattgttaac atggatgtta     3840
   cagctcaaaa gatttataaa agattttaac ctattttctc ccttattatc cactgctaat     3900
   gtggatgtat gttcaaacac cttttagtat tgatagctta catatggcca aaggaataca     3960
   gtttatagca aaacatgggt atgctgtagc taactttata aaagtgtaat ataacaatgt     4020
   aaaaaattat atatctggga ggattttttg gttgcctaaa gtggctatag ttactgattt     4080
   tttattatgt aagcaaaacc aataaaaatt taagtttttt taacaactac cttatttttc     4140
   actgtacaga cactaattca ttaaatacta attgattgtt taaaagaaat ataaatgtga     4200
   caagtggaca ttatttatgt taaatataca attatcaagc aagtatgaag ttattcaatt     4260
   aaaatgccac atttctggtc tctggg                                           4286
<210> 6
   <211> 436
   <212> PRT
   <213> Homo sapiens
<400> 6
<210> 7
   <211> 8091
   <212> DNA
   <213> Homo sapiens
<400> 7
   acgcggcgcg gaggctggcc cgggacgcgc ccggagccca gggaaggagg gaggagggga     60
   gggtcgcggc cggccgccat ggggccgggg gcccgtggcc gccgccgccg ccgtcgcccg    120
   atgtcgccgc caccgccacc gccacccgtg cgggcgctgc ccctgctgct gctgctagcg    180
   gggccggggg ctgcagcccc cccttgcctg gacggaagcc cgtgtgcaaa tggaggtcgt    240
   tgcacccagc tgccctcccg ggaggctgcc tgcctgtgcc cgcctggctg ggtgggtgag    300
   cggtgtcagc tggaggaccc ctgtcactca ggcccctgtg ctggccgtgg tgtctgccag    360
   agttcagtgg tggctggcac cgcccgattc tcatgccggt gcccccgtgg cttccgaggc    420
   cctgactgct ccctgccaga tccctgcctc agcagccctt gtgcccacgg tgcccgctgc    480
   tcagtggggc ccgatggacg cttcctctgc tcctgcccac ctggctacca gggccgcagc    540
   tgccgaagcg acgtggatga gtgccgggtg ggtgagccct gccgccatgg tggcacctgc    600
   ctcaacacac ctggctcctt ccgctgccag tgtccagctg gctacacagg gccactatgt    660
   gagaaccccg cggtgccctg tgcgccctca ccatgccgta acgggggcac ctgcaggcag    720
   agtggcgacc tcacttacga ctgtgcctgt cttcctgggt ttgagggtca gaattgtgaa    780
   gtgaacgtgg acgactgtcc aggacaccga tgtctcaatg gggggacatg cgtggatggc    840
   gtcaacacct ataactgcca gtgccctcct gagtggacag gccagttctg cacggaggac    900
   gtggatgagt gtcagctgca gcccaacgcc tgccacaatg ggggtacctg cttcaacacg    960
   ctgggtggcc acagctgcgt gtgtgtcaat ggctggacag gtgagagctg cagtcagaat   1020
   atcgatgact gtgccacagc cgtgtgcttc catggggcca cctgccatga ccgcgtggct   1080
   tctttctact gtgcctgccc catgggcaag actggcctcc tgtgtcacct ggatgacgcc   1140
   tgtgtcagca acccctgcca cgaggatgct atctgtgaca caaatccggt gaacggccgg   1200
   gccatttgca cctgtcctcc cggcttcacg ggtggggcat gtgaccagga tgtggacgag   1260
   tgctctatcg gcgccaaccc ctgcgagcac ttgggcaggt gcgtgaacac gcagggctcc   1320
   ttcctgtgcc agtgcggtcg tggctacact ggacctcgct gtgagaccga tgtcaacgag   1380
   tgtctgtcgg ggccctgccg aaaccaggcc acgtgcctcg accgcatagg ccagttcacc   1440
   tgtatctgta tggcaggctt cacaggaacc tattgcgagg tggacattga cgagtgtcag   1500
   agtagcccct gtgtcaacgg tggggtctgc aaggaccgag tcaatggctt cagctgcacc    1560
   tgcccctcgg gcttcagcgg ctccacgtgt cagctggacg tggacgaatg cgccagcacg    1620
   ccctgcagga atggcgccaa atgcgtggac cagcccgatg gctacgagtg ccgctgtgcc    1680
   gagggctttg agggcacgct gtgtgatcgc aacgtggacg actgctcccc tgacccatgc    1740
   caccatggtc gctgcgtgga tggcatcgcc agcttctcat gtgcctgtgc tcctggctac    1800
   acgggcacac gctgcgagag ccaggtggac gaatgccgca gccagccctg ccgccatggc    1860
   ggcaaatgcc tagacctggt ggacaagtac ctctgccgct gcccttctgg gaccacaggt    1920
   gtgaactgcg aagtgaacat tgacgactgt gccagcaacc cctgcacctt tggagtctgc    1980
   cgtgatggca tcaaccgcta cgactgtgtc tgccaacctg gcttcacagg gcccctttgt    2040
   aacgtggaga tcaatgagtg tgcttccagc ccatgcggcg agggaggttc ctgtgtggat    2100
   ggggaaaatg gcttccgctg cctctgcccg cctggctcct tgcccccact ctgcctcccc   2160
   ccgagccatc cctgtgccca tgagccctgc agtcacggca tctgctatga tgcacctggc    2220
   gggttccgct gtgtgtgtga gcctggctgg agtggccccc gctgcagcca gagcctggcc    2280
   cgagacgcct gtgagtccca gccgtgcagg gccggtggga catgcagcag cgatggaatg    2340
   ggtttccact gcacctgccc gcctggtgtc cagggacgtc agtgtgaact cctctccccc    2400
   tgcaccccga acccctgtga gcatgggggc cgctgcgagt ctgcccctgg ccagctgcct    2460
   gtctgctcct gcccccaggg ctggcaaggc ccacgatgcc agcaggatgt ggacgagtgt    2520
   gctggccccg caccctgtgg ccctcatggt atctgcacca acctggcagg gagtttcagc    2580
   tgcacctgcc atggagggta cactggccct tcctgtgatc aggacatcaa tgactgtgac    2640
   cccaacccat gcctgaacgg tggctcgtgc caagacggcg tgggctcctt ttcctgctcc    2700
   tgcctccctg gtttcgccgg cccacgatgc gcccgcgatg tggatgagtg cctgagcaac    2760
   ccctgcggcc cgggcacctg taccgaccac gtggcctcct tcacctgcac ctgcccgccg    2820
   ggctacggag gcttccactg cgaacaggac ctgcccgact gcagccccag ctcctgcttc    2880
   aatggcggga cctgtgtgga cggcgtgaac tcgttcagct gcctgtgccg tcccggctac    2940
   acaggagccc actgccaaca tgaggcagac ccctgcctct cgcggccctg cctacacggg    3000
   ggcgtctgca gcgccgccca ccctggcttc cgctgcacct gcctcgagag cttcacgggc    3060
   ccgcagtgcc agacgctggt ggattggtgc agccgccagc cttgtcaaaa cgggggtcgc    3120
   tgcgtccaga ctggggccta ttgcctttgt ccccctggat ggagcggacg cctctgtgac    3180
   atccgaagct tgccctgcag ggaggccgca gcccagatcg gggtgcggct ggagcagctg    3240
   tgtcaggcgg gtgggcagtg tgtggatgaa gacagctccc actactgcgt gtgcccagag    3300
   ggccgtactg gtagccactg tgagcaggag gtggacccct gcttggccca gccctgccag    3360
   catgggggga cctgccgtgg ctatatgggg ggctacatgt gtgagtgtct tcctggctac    3420
   aatggtgata actgtgagga cgacgtggac gagtgtgcct cccagccctg ccagcacggg    3480
   ggttcatgca ttgacctcgt ggcccgctat ctctgctcct gtcccccagg aacgctgggg    3540
   gtgctctgcg agattaatga ggatgactgc ggcccaggcc caccgctgga ctcagggccc    3600
   cggtgcctac acaatggcac ctgcgtggac ctggtgggtg gtttccgctg cacctgtccc    3660
   ccaggataca ctggtttgcg ctgcgaggca gacatcaatg agtgtcgctc aggtgcctgc    3720
   cacgcggcac acacccggga ctgcctgcag gacccaggcg gaggtttccg ttgcctttgt    3780
   catgctggct tctcaggtcc tcgctgtcag actgtcctgt ctccctgcga gtcccagcca    3840
   tgccagcatg gaggccagtg ccgtcctagc ccgggtcctg ggggtgggct gaccttcacc    3900
   tgtcactgtg cccagccgtt ctggggtccg cgttgcgagc gggtggcgcg ctcctgccgg    3960
   gagctgcagt gcccggtggg cgtcccatgc cagcagacgc cccgcgggcc gcgctgcgcc    4020
   tgccccccag ggttgtcggg accctcctgc cgcagcttcc cggggtcgcc gccgggggcc    4080
   agcaacgcca gctgcgcggc cgccccctgt ctccacgggg gctcctgccg ccccgcgccg    4140
   ctcgcgccct tcttccgctg cgcttgcgcg cagggctgga ccgggccgcg ctgcgaggcg    4200
   cccgccgcgg cacccgaggt ctcggaggag ccgcggtgcc cgcgcgccgc ctgccaggcc    4260
   aagcgcgggg accagcgctg cgaccgcgag tgcaacagcc caggctgcgg ctgggacggc    4320
   ggcgactgct cgctgagcgt gggcgacccc tggcggcaat gcgaggcgct gcagtgctgg    4380
   cgcctcttca acaacagccg ctgcgacccc gcctgcagct cgcccgcctg cctctacgac    4440
   aacttcgact gccacgccgg tggccgcgag cgcacttgca acccggtgta cgagaagtac    4500
   tgcgccgacc actttgccga cggccgctgc gaccagggct gcaacacgga ggagtgcggc    4560
   tgggatgggc tggattgtgc cagcgaggtg ccggccctgc tggcccgcgg cgtgctggtg    4620
   ctcacagtgc tgctgccgcc ggaggagcta ctgcgttcca gcgccgactt tctgcagcgg    4680
   ctcagcgcca tcctgcgcac ctcgctgcgc ttccgcctgg acgcgcacgg ccaggccatg    4740
   gtcttccctt accaccggcc tagtcctggc tccgaacccc gggcccgtcg ggagctggcc    4800
   cccgaggtga tcggctcggt agtaatgctg gagattgaca accggctctg cctgcagtcg    4860
   cctgagaatg atcactgctt ccccgatgcc cagagcgccg ctgactacct gggagcgttg    4920
   tcagcggtgg agcgcctgga cttcccgtac ccactgcggg acgtgcgggg ggagccgctg    4980
   gagcctccag aacccagcgt cccgctgctg ccactgctag tggcgggcgc tgtcttgctg    5040
   ctggtcattc tcgtcctggg tgtcatggtg gcccggcgca agcgcgagca cagcaccctc    5100
   tggttccctg agggcttctc actgcacaag gacgtggcct ctggtcacaa gggccggcgg    5160
   gaacccgtgg gccaggacgc gctgggcatg aagaacatgg ccaagggtga gagcctgatg    5220
   ggggaggtgg ccacagactg gatggacaca gagtgcccag aggccaagcg gctaaaggta    5280
   gaggagccag gcatgggggc tgaggaggct gtggattgcc gtcagtggac tcaacaccat    5340
   ctggttgctg ctgacatccg cgtggcacca gccatggcac tgacaccacc acagggcgac    5400
   gcagatgctg atggcatgga tgtcaatgtg cgtggcccag atggcttcac cccgctaatg    5460
   ctggcttcct tctgtggggg ggctctggag ccaatgccaa ctgaagagga tgaggcagat    5520
   gacacatcag ctagcatcat ctccgacctg atctgccagg gggctcagct tggggcacgg    5580
   actgaccgta ctggcgagac tgctttgcac ctggctgccc gttatgcccg tgctgatgca    5640
   gccaagcggc tgctggatgc tggggcagac accaatgccc aggaccactc aggccgcact     5700
   cccctgcaca cagctgtcac agccgatgcc cagggtgtct tccagattct catccgaaac     5760
   cgctctacag acttggatgc ccgcatggca gatggctcaa cggcactgat cctggcggcc     5820
   cgcctggcag tagagggcat ggtggaagag ctcatcgcca gccatgctga tgtcaatgct     5880
   gtggatgagc ttgggaaatc agccttacac tgggctgcgg ctgtgaacaa cgtggaagcc     5940
   actttggccc tgctcaaaaa tggagccaat aaggacatgc aggatagcaa ggaggagacc     6000
   cccctattcc tggccgcccg cgagggcagc tatgaggctg ccaagctgct gttggaccac     6060
   tttgccaacc gtgagatcac cgaccacctg gacaggctgc cgcgggacgt agcccaggag     6120
   agactgcacc aggacatcgt gcgcttgctg gatcaaccca gtgggccccg cagccccccc     6180
   ggtccccacg gcctggggcc tctgctctgt cctccagggg ccttcctccc tggcctcaaa     6240
   gcggcacagt cggggtccaa gaagagcagg aggccccccg ggaaggcggg gctggggccg     6300
   caggggcccc gggggcgggg caagaagctg acgctggcct gcccgggccc cctggctgac     6360
   agctcggtca cgctgtcgcc cgtggactcg ctggactccc cgcggccttt cggtgggccc     6420
   cctgcttccc ctggtggctt cccccttgag gggccctatg cagctgccac tgccactgca     6480
   gtgtctctgg cacagcttgg tggcccaggc cgggcaggtc tagggcgcca gccccctgga     6540
   ggatgtgtac tcagcctggg cctgctgaac cctgtggctg tgcccctcga ttgggcccgg     6600
   ctgcccccac ctgcccctcc aggcccctcg ttcctgctgc cactggcgcc gggaccccag     6660
   ctgctcaacc cagggacccc cgtctccccg caggagcggc cccgccttta cctggcagtc     6720
   ccaggacatg gcgaggagta cccggtggct ggggcacaca gcagcccccc aaaggcccgc     6780
   ttcctgcggg ttcccagtga gcacccttac ctgaccccat cccccgaatc ccctgagcac     6840
   tgggccagcc cctcacctcc ctccctctca gactggtccg aatccacgcc tagcccagcc     6900
   actgccactg gggccatggc caccaccact ggggcactgc ctgcccagcc acttcccttg     6960
   tctgttccca gctcccttgc tcaggcccag acccagctgg ggccccagcc ggaagttacc     7020
   cccaagaggc aagtgttggc ctgagacgct cgtcagttct tagatcttgg gggcctaaag     7080
   agacccccgt cctgcctcct ttctttctct gtctcttcct tccttttagt ctttttcatc     7140
   ctcttctctt tccaccaacc ctcctgcatc cttgccttgc agcgtgaccg agataggtca     7200
   tcagcccagg gcttcagtct tcctttattt ataatgggtg ggggctacca cccaccctct     7260
   cagtcttgtg aagagtctgg gacctccttc ttccccactt ctctcttccc tcattccttt     7320
   ctctctcctt ctggcctctc atttccttac actctgacat gaatgaatta ttattatttt     7380
   tctttttctt ttttttttta cattttgtat agaaacaaat tcatttaaac aaacttatta     7440
   ttattatttt ttacaaaata tatatatgga gatgctccct ccccctgtga accccccagt     7500
   gcccccgtgg ggctgagtct gtgggcccat tcggccaagc tggattctgt gtacctagta     7560
   cacaggcatg actgggatcc cgtgtaccga gtacacgacc caggtatgta ccaagtaggc     7620
   acccttgggc gcacccactg gggccagggg tcgggggagt gttgggagcc tcctccccac     7680
   cccacctccc tcacttcact gcattccaga ttggacatgt tccatagcct tgctggggaa     7740
   gggcccactg ccaactccct ctgccccagc cccacccttg gccatctccc tttgggaact     7800
   agggggctgc tggtgggaaa tgggagccag ggcagatgta tgcattcctt tatgtccctg     7860
   taaatgtggg actacaagaa gaggagctgc ctgagtggta ctttctcttc ctggtaatcc     7920
   tctggcccag ccttatggca gaatagaggt atttttaggc tatttttgta atatggcttc     7980
   tggtcaaaat ccctgtgtag ctgaattccc aagccctgca ttgtacagcc ccccactccc     8040
   ctcaccacct aataaaggaa tagttaacac tcaaaaaaaa aaaaaaaaaa a              8091
<210> 8
   <211> 2321
   <212> PRT
   <213> Homo sapiens
<400> 8
   Met Gly Pro Gly Ala Arg Gly Arg Arg Arg Arg Arg Arg Pro Met Ser
    1              5                   10                  15
   Pro Pro Pro Pro Pro Pro Pro Val Arg Ala Leu Pro Leu Leu Leu Leu
               20                  25                  30
   Leu Ala Gly Pro Gly Ala Ala Ala Pro Pro Cys Leu Asp Gly Ser Pro
           35                  40                  45
   Cys Ala Asn Gly Gly Arg Cys Thr Gln Leu Pro Ser Arg Glu Ala Ala
       50                  55                  60
   Cys Leu Cys Pro Pro Gly Trp Val Gly Glu Arg Cys Gln Leu Glu Asp
   65                  70                  75                  80
   Pro Cys His Ser Gly Pro Cys Ala Gly Arg Gly Val Cys Gln Ser Ser
                   85                  90                  95
   Val Val Ala Gly Thr Ala Arg Phe Ser Cys Arg Cys Pro Arg Gly Phe
               100                 105                 110
   Arg Gly Pro Asp Cys Ser Leu Pro Asp Pro Cys Leu Ser Ser Pro Cys
           115                 120                 125
   Ala His Gly Ala Arg Cys Ser Val Gly Pro Asp Gly Arg Phe Leu Cys
       130                 135                 140
   Ser Cys Pro Pro Gly Tyr Gln Gly Arg Ser Cys Arg Ser Asp Val Asp
   145                 150                 155                 160
   Glu Cys Arg Val Gly Glu Pro Cys Arg His Gly Gly Thr Cys Leu Asn
                   165                 170                 175
   Thr Pro Gly Ser Phe Arg Cys Gln Cys Pro Ala Gly Tyr Thr Gly Pro
               180                 185                 190
   Leu Cys Glu Asn Pro Ala Val Pro Cys Ala Pro Ser Pro Cys Arg Asn
           195                 200                 205
   Gly Gly Thr Cys Arg Gln Ser Gly Asp Leu Thr Tyr Asp Cys Ala Cys
       210                 215                 220
   Leu Pro Gly Phe Glu Gly Gln Asn Cys Glu Val Asn Val Asp Asp Cys
   225                 230                 235                 240
   Pro Gly His Arg Cys Leu Asn Gly Gly Thr Cys Val Asp Gly Val Asn
                   245                 250                 255
   Thr Tyr Asn Cys Gln Cys Pro Pro Glu Trp Thr Gly Gln Phe Cys Thr
               260                 265                 270
   Glu Asp Val Asp Glu Cys Gln Leu Gln Pro Asn Ala Cys His Asn Gly
           275                 280                 285
   Gly Thr Cys Phe Asn Thr Leu Gly Gly His Ser Cys Val Cys Val Asn
       290                 295                 300
   Gly Trp Thr Gly Glu Ser Cys Ser Gln Asn Ile Asp Asp Cys Ala Thr
   305                 310                 315                 320
   Ala Val Cys Phe His Gly Ala Thr Cys His Asp Arg Val Ala Ser Phe
                   325                 330                 335
   Tyr Cys Ala Cys Pro Met Gly Lys Thr Gly Leu Leu Cys His Leu Asp
               340                 345                 350
   Asp Ala Cys Val Ser Asn Pro Cys His Glu Asp Ala Ile Cys Asp Thr
           355                 360                 365
   Asn Pro Val Asn Gly Arg Ala Ile Cys Thr Cys Pro Pro Gly Phe Thr
       370                 375                 380
   Gly Gly Ala Cys Asp Gln Asp Val Asp Glu Cys Ser Ile Gly Ala Asn
   385                 390                 395                 400
   Pro Cys Glu His Leu Gly Arg Cys Val Asn Thr Gln Gly Ser Phe Leu
                   405                 410                 415
   Cys Gln Cys Gly Arg Gly Tyr Thr Gly Pro Arg Cys Glu Thr Asp Val
               420                 425                 430
   Asn Glu Cys Leu Ser Gly Pro Cys Arg Asn Gln Ala Thr Cys Leu Asp
           435                 440                 445
   Arg Ile Gly Gln Phe Thr Cys Ile Cys Met Ala Gly Phe Thr Gly Thr
       450                 455                 460
   Tyr Cys Glu Val Asp Ile Asp Glu Cys Gln Ser Ser Pro Cys Val Asn
   465                 470                 475                 480
   Gly Gly Val Cys Lys Asp Arg Val Asn Gly Phe Ser Cys Thr Cys Pro
                   485                 490                 495
   Ser Gly Phe Ser Gly Ser Thr Cys Gln Leu Asp Val Asp Glu Cys Ala
               500                 505                 510
   Ser Thr Pro Cys Arg Asn Gly Ala Lys Cys Val Asp Gln Pro Asp Gly
           515                 520                 525
   Tyr Glu Cys Arg Cys Ala Glu Gly Phe Glu Gly Thr Leu Cys Asp Arg
       530                 535                 540
   Asn Val Asp Asp Cys Ser Pro Asp Pro Cys His His Gly Arg Cys Val
   545                 550                 555                 560
   Asp Gly Ile Ala Ser Phe Ser Cys Ala Cys Ala Pro Gly Tyr Thr Gly
                   565                 570                 575
   Thr Arg Cys Glu Ser Gln Val Asp Glu Cys Arg Ser Gln Pro Cys Arg
               580                 585                 590
   His Gly Gly Lys Cys Leu Asp Leu Val Asp Lys Tyr Leu Cys Arg Cys
           595                 600                 605
   Pro Ser Gly Thr Thr Gly Val Asn Cys Glu Val Asn Ile Asp Asp Cys
       610                 615                 620
   Ala Ser Asn Pro Cys Thr Phe Gly Val Cys Arg Asp Gly Ile Asn Arg
   625                 630                 635                 640
   Tyr Asp Cys Val Cys Gln Pro Gly Phe Thr Gly Pro Leu Cys Asn Val
                   645                 650                 655
   Glu Ile Asn Glu Cys Ala Ser Ser Pro Cys Gly Glu Gly Gly Ser Cys
               660                 665                 670
   Val Asp Gly Glu Asn Gly Phe Arg Cys Leu Cys Pro Pro Gly Ser Leu
           675                 680                 685
   Pro Pro Leu Cys Leu Pro Pro Ser His Pro Cys Ala His Glu Pro Cys
       690                 695                 700
   Ser His Gly Ile Cys Tyr Asp Ala Pro Gly Gly Phe Arg Cys Val Cys
   705                 710                 715                 720
   Glu Pro Gly Trp Ser Gly Pro Arg Cys Ser Gln Ser Leu Ala Arg Asp
                   725                 730                 735
   Ala Cys Glu Ser Gln Pro Cys Arg Ala Gly Gly Thr Cys Ser Ser Asp
               740                 745                 750
   Gly Met Gly Phe His Cys Thr Cys Pro Pro Gly Val Gln Gly Arg Gln
           755                 760                 765
   Cys Glu Leu Leu Ser Pro Cys Thr Pro Asn Pro Cys Glu His Gly Gly
       770                 775                 780
   Arg Cys Glu Ser Ala Pro Gly Gln Leu Pro Val Cys Ser Cys Pro Gln
   785                 790                 795                 800
   Gly Trp Gln Gly Pro Arg Cys Gln Gln Asp Val Asp Glu Cys Ala Gly
                   805                 810                 815
   Pro Ala Pro Cys Gly Pro His Gly Ile Cys Thr Asn Leu Ala Gly Ser
               820                 825                 830
   Phe Ser Cys Thr Cys His Gly Gly Tyr Thr Gly Pro Ser Cys Asp Gln
           835                 840                 845
   Asp Ile Asn Asp Cys Asp Pro Asn Pro Cys Leu Asn Gly Gly Ser Cys
       850                 855                 860
   Gln Asp Gly Val Gly Ser Phe Ser Cys Ser Cys Leu Pro Gly Phe Ala
   865                 870                 875                 880
   Gly Pro Arg Cys Ala Arg Asp Val Asp Glu Cys Leu Ser Asn Pro Cys
                   885                 890                 895
   Gly Pro Gly Thr Cys Thr Asp His Val Ala Ser Phe Thr Cys Thr Cys
               900                 905                 910
   Pro Pro Gly Tyr Gly Gly Phe His Cys Glu Gln Asp Leu Pro Asp Cys
           915                 920                 925
   Ser Pro Ser Ser Cys Phe Asn Gly Gly Thr Cys Val Asp Gly Val Asn
       930                 935                 940
   Ser Phe Ser Cys Leu Cys Arg Pro Gly Tyr Thr Gly Ala His Cys Gln
   945                 950                 955                 960
   His Glu Ala Asp Pro Cys Leu Ser Arg Pro Cys Leu His Gly Gly Val
                   965                 970                 975
   Cys Ser Ala Ala His Pro Gly Phe Arg Cys Thr Cys Leu Glu Ser Phe
               980                 985                 990
   Thr Gly Pro Gln Cys Gln Thr Leu Val Asp Trp Cys Ser Arg Gln Pro
           995                 1000                1005
   Cys Gln Asn Gly Gly Arg Cys Val Gln Thr Gly Ala Tyr Cys Leu Cys
       1010                1015                1020
   Pro Pro Gly Trp Ser Gly Arg Leu Cys Asp Ile Arg Ser Leu Pro Cys
   1025                1030                1035                1040
   Arg Glu Ala Ala Ala Gln Ile Gly Val Arg Leu Glu Gln Leu Cys Gln
                   1045                1050                1055
   Ala Gly Gly Gln Cys Val Asp Glu Asp Ser Ser His Tyr Cys Val Cys
               1060                1065                1070
   Pro Glu Gly Arg Thr Gly Ser His Cys Glu Gln Glu Val Asp Pro Cys
           1075                1080                1085
   Leu Ala Gln Pro Cys Gln His Gly Gly Thr Cys Arg Gly Tyr Met Gly
       1090                1095                1100
   Gly Tyr Met Cys Glu Cys Leu Pro Gly Tyr Asn Gly Asp Asn Cys Glu
   1105                1110                1115                1120
   Asp Asp Val Asp Glu Cys Ala Ser Gln Pro Cys Gln His Gly Gly Ser
                   1125                1130                1135
   Cys Ile Asp Leu Val Ala Arg Tyr Leu Cys Ser Cys Pro Pro Gly Thr
               1140                1145                1150
   Leu Gly Val Leu Cys Glu Ile Asn Glu Asp Asp Cys Gly Pro Gly Pro
           1155                1160                1165
   Pro Leu Asp Ser Gly Pro Arg Cys Leu His Asn Gly Thr Cys Val Asp
       1170                1175                1180
   Leu Val Gly Gly Phe Arg Cys Thr Cys Pro Pro Gly Tyr Thr Gly Leu
   1185                1190                1195                1200
   Arg Cys Glu Ala Asp Ile Asn Glu Cys Arg Ser Gly Ala Cys His Ala
                   1205                1210                1215
   Ala His Thr Arg Asp Cys Leu Gln Asp Pro Gly Gly Gly Phe Arg Cys
               1220                1225                1230
   Leu Cys His Ala Gly Phe Ser Gly Pro Arg Cys Gln Thr Val Leu Ser
           1235                1240                1245
   Pro Cys Glu Ser Gln Pro Cys Gln His Gly Gly Gln Cys Arg Pro Ser
       1250                1255                1260
   Pro Gly Pro Gly Gly Gly Leu Thr Phe Thr Cys His Cys Ala Gln Pro
   1265                1270                1275                1280
   Phe Trp Gly Pro Arg Cys Glu Arg Val Ala Arg Ser Cys Arg Glu Leu
                   1285                1290                1295
   Gln Cys Pro Val Gly Val Pro Cys Gln Gln Thr Pro Arg Gly Pro Arg
               1300                1305                1310
   Cys Ala Cys Pro Pro Gly Leu Ser Gly Pro Ser Cys Arg Ser Phe Pro
           1315                1320                1325
   Gly Ser Pro Pro Gly Ala Ser Asn Ala Ser Cys Ala Ala Ala Pro Cys
       1330                1335                1340
   Leu His Gly Gly Ser Cys Arg Pro Ala Pro Leu Ala Pro Phe Phe Arg
   1345                1350                1355                1360
   Cys Ala Cys Ala Gln Gly Trp Thr Gly Pro Arg Cys Glu Ala Pro Ala
                   1365                1370                1375
   Ala Ala Pro Glu Val Ser Glu Glu Pro Arg Cys Pro Arg Ala Ala Cys
               1380                1385                1390
   Gln Ala Lys Arg Gly Asp Gln Arg Cys Asp Arg Glu Cys Asn Ser Pro
           1395                1400                1405
   Gly Cys Gly Trp Asp Gly Gly Asp Cys Ser Leu Ser Val Gly Asp Pro
       1410                1415                1420
   Trp Arg Gln Cys Glu Ala Leu Gln Cys Trp Arg Leu Phe Asn Asn Ser
   1425                1430                1435                1440
   Arg Cys Asp Pro Ala Cys Ser Ser Pro Ala Cys Leu Tyr Asp Asn Phe
                   1445                1450                1455
   Asp Cys His Ala Gly Gly Arg Glu Arg Thr Cys Asn Pro Val Tyr Glu
               1460                1465                1470
   Lys Tyr Cys Ala Asp His Phe Ala Asp Gly Arg Cys Asp Gln Gly Cys
           1475                1480                1485
   Asn Thr Glu Glu Cys Gly Trp Asp Gly Leu Asp Cys Ala Ser Glu Val
       1490                1495                1500
   Pro Ala Leu Leu Ala Arg Gly Val Leu Val Leu Thr Val Leu Leu Pro
   1505                1510                1515                1520
   Pro Glu Glu Leu Leu Arg Ser Ser Ala Asp Phe Leu Gln Arg Leu Ser
                   1525                1530                1535
   Ala Ile Leu Arg Thr Ser Leu Arg Phe Arg Leu Asp Ala His Gly Gln
               1540                1545                1550
   Ala Met Val Phe Pro Tyr His Arg Pro Ser Pro Gly Ser Glu Pro Arg
           1555                1560                1565
   Ala Arg Arg Glu Leu Ala Pro Glu Val Ile Gly Ser Val Val Met Leu
       1570                1575                1580
   Glu Ile Asp Asn Arg Leu Cys Leu Gln Ser Pro Glu Asn Asp His Cys
   1585                1590                1595                1600
   Phe Pro Asp Ala Gln Ser Ala Ala Asp Tyr Leu Gly Ala Leu Ser Ala
                   1605                1610                1615
   Val Glu Arg Leu Asp Phe Pro Tyr Pro Leu Arg Asp Val Arg Gly Glu
               1620                1625                1630
   Pro Leu Glu Pro Pro Glu Pro Ser Val Pro Leu Leu Pro Leu Leu Val
           1635                1640                1645
   Ala Gly Ala Val Leu Leu Leu Val Ile Leu Val Leu Gly Val Met Val
       1650                1655                1660
   Ala Arg Arg Lys Arg Glu His Ser Thr Leu Trp Phe Pro Glu Gly Phe
   1665                1670                1675                1680
   Ser Leu His Lys Asp Val Ala Ser Gly His Lys Gly Arg Arg Glu Pro
                   1685                1690                1695
   Val Gly Gln Asp Ala Leu Gly Met Lys Asn Met Ala Lys Gly Glu Ser
               1700                1705                1710
   Leu Met Gly Glu Val Ala Thr Asp Trp Met Asp Thr Glu Cys Pro Glu
           1715                1720                1725
   Ala Lys Arg Leu Lys Val Glu Glu Pro Gly Met Gly Ala Glu Glu Ala
       1730                1735                1740
   Val Asp Cys Arg Gln Trp Thr Gln His His Leu Val Ala Ala Asp Ile
   1745                1750                1755                1760
   Arg Val Ala Pro Ala Met Ala Leu Thr Pro Pro Gln Gly Asp Ala Asp
                   1765                1770                1775
   Ala Asp Gly Met Asp Val Asn Val Arg Gly Pro Asp Gly Phe Thr Pro
               1780                1785                1790
   Leu Met Leu Ala Ser Phe Cys Gly Gly Ala Leu Glu Pro Met Pro Thr
           1795                1800                1805
   Glu Glu Asp Glu Ala Asp Asp Thr Ser Ala Ser Ile Ile Ser Asp Leu
       1810                1815                1820
   Ile Cys Gln Gly Ala Gln Leu Gly Ala Arg Thr Asp Arg Thr Gly Glu
   1825                1830                1835                1840
   Thr Ala Leu His Leu Ala Ala Arg Tyr Ala Arg Ala Asp Ala Ala Lys
                   1845                1850                1855
   Arg Leu Leu Asp Ala Gly Ala Asp Thr Asn Ala Gln Asp His Ser Gly
               1860                1865                1870
   Arg Thr Pro Leu His Thr Ala Val Thr Ala Asp Ala Gln Gly Val Phe
           1875                1880                1885
   Gln Ile Leu Ile Arg Asn Arg Ser Thr Asp Leu Asp Ala Arg Met Ala
       1890                1895                1900
   Asp Gly Ser Thr Ala Leu Ile Leu Ala Ala Arg Leu Ala Val Glu Gly
   1905                1910                1915                1920
   Met Val Glu Glu Leu Ile Ala Ser His Ala Asp Val Asn Ala Val Asp
                   1925                1930                1935
   Glu Leu Gly Lys Ser Ala Leu His Trp Ala Ala Ala Val Asn Asn Val
               1940                1945                1950
   Glu Ala Thr Leu Ala Leu Leu Lys Asn Gly Ala Asn Lys Asp Met Gln
           1955                1960                1965
   Asp Ser Lys Glu Glu Thr Pro Leu Phe Leu Ala Ala Arg Glu Gly Ser
       1970                1975                1980
   Tyr Glu Ala Ala Lys Leu Leu Leu Asp His Phe Ala Asn Arg Glu Ile
   1985                1990                1995                2000
   Thr Asp His Leu Asp Arg Leu Pro Arg Asp Val Ala Gln Glu Arg Leu
                   2005                2010                2015
   His Gln Asp Ile Val Arg Leu Leu Asp Gln Pro Ser Gly Pro Arg Ser
               2020                2025                2030
   Pro Pro Gly Pro His Gly Leu Gly Pro Leu Leu Cys Pro Pro Gly Ala
           2035                2040                2045
   Phe Leu Pro Gly Leu Lys Ala Ala Gln Ser Gly Ser Lys Lys Ser Arg
       2050                2055                2060
   Arg Pro Pro Gly Lys Ala Gly Leu Gly Pro Gln Gly Pro Arg Gly Arg
   2065                2070                2075                2080
   Gly Lys Lys Leu Thr Leu Ala Cys Pro Gly Pro Leu Ala Asp Ser Ser
                   2085                2090                2095
   Val Thr Leu Ser Pro Val Asp Ser Leu Asp Ser Pro Arg Pro Phe Gly
               2100                2105                2110
   Gly Pro Pro Ala Ser Pro Gly Gly Phe Pro Leu Glu Gly Pro Tyr Ala
           2115                2120                2125
   Ala Ala Thr Ala Thr Ala Val Ser Leu Ala Gln Leu Gly Gly Pro Gly
       2130                2135                2140
   Arg Ala Gly Leu Gly Arg Gln Pro Pro Gly Gly Cys Val Leu Ser Leu
   2145                2150                2155                2160
   Gly Leu Leu Asn Pro Val Ala Val Pro Leu Asp Trp Ala Arg Leu Pro
                   2165                2170                2175
   Pro Pro Ala Pro Pro Gly Pro Ser Phe Leu Leu Pro Leu Ala Pro Gly
               2180                2185                2190
   Pro Gln Leu Leu Asn Pro Gly Thr Pro Val Ser Pro Gln Glu Arg Pro
           2195                2200                2205
   Pro Pro Tyr Leu Ala Val Pro Gly His Gly Glu Glu Tyr Pro Val Ala
       2210                2215                2220
   Gly Ala His Ser Ser Pro Pro Lys Ala Arg Phe Leu Arg Val Pro Ser
   2225                2230                2235                2240
   Glu His Pro Tyr Leu Thr Pro Ser Pro Glu Ser Pro Glu His Trp Ala
                   2245                2250                2255
   Ser Pro Ser Pro Pro Ser Leu Ser Asp Trp Ser Glu Ser Thr Pro Ser
               2260                2265                2270
   Pro Ala Thr Ala Thr Gly Ala Met Ala Thr Thr Thr Gly Ala Leu Pro
           2275                2280                2285
   Ala Gln Pro Leu Pro Leu Ser Val Pro Ser Ser Leu Ala Gln Ala Gln
       2290                2295                2300
   Thr Gln Leu Gly Pro Gln Pro Glu Val Thr Pro Lys Arg Gln Val Leu
   2305                2310                2315                2320
   Ala
<210> 9
   <211> 1638
   <212> DNA
   <213> Homo sapiens
<400> 9
   cgggccctgc gggcgcgggg ctgaaggcgg aaccacgacg ggcagagagc acggagccgg      60
   gaagcccctg ggcgcccgtc ggagggctat ggagcagcgg ccgcggggct gcgcggcggt     120
   ggcggcggcg ctcctcctgg tgctgctggg ggcccgggcc cagggcggca ctcgtagccc     180
   caggtgtgac tgtgccggtg acttccacaa gaagattggt ctgttttgtt gcagaggctg     240
   cccagcgggg cactacctga aggccccttg cacggagccc tgcggcaact ccacctgcct     300
   tgtgtgtccc caagacacct tcttggcctg ggagaaccac cataattctg aatgtgcccg     360
   ctgccaggcc tgtgatgagc aggcctccca ggtggcgctg gagaactgtt cagcagtggc     420
   cgacacccgc tgtggctgta agccaggctg gtttgtggag tgccaggtca gccaatgtgt     480
   cagcagttca cccttctact gccaaccatg cctagactgc ggggccctgc accgccacac     540
   acggctactc tgttcccgca gagatactga ctgtgggacc tgcctgcctg gcttctatga     600
   acatggcgat ggctgcgtgt cctgccccac gagcaccctg ggagctgtcc cagagcgctg      660
   tgccgctgtc tgtggctgga ggcagatgtt ctgggtccag gtgctcctgg ctggccttgt     720
   ggtccccctc ctgcttgggg ccaccctgac ctacacatac cgccactgct ggcctcacaa      780
   gcccctggtt actgcagatg aagctgggat ggaggctctg accccaccac cggccaccca     840
   tctgtcaccc ttggacagcg cccacaccct tctagcacct cctgacagca gtgagaagat     900
   ctgcaccgtc cagttggtgg gtaacagctg gacccctggc taccccgaga cccaggaggc     960
   gctctgcccg caggtgacat ggtcctggga ccagttgccc agcagagctc ttggccccgc    1020
   tgctgcgccc acactctcgc cagagtcccc agccggctcg ccagccatga tgctgcagcc    1080
   gggcccgcag ctctacgacg tgatggacgc ggtcccagcg cggcgctgga aggagttcgt    1140
   gcgcacgctg gggctgcgcg aggcagagat cgaagccgtg gaggtggaga tcggccgctt    1200
   ccgagaccag cagtacgaga tgctcaagcg ctggcgccag cagcagcccg cgggcctcgg    1260
   agccgtttac gcggccctgg agcgcatggg gctggacggc tgcgtggaag acttgcgcag    1320
   ccgcctgcag cgcggcccgt gacacggcgc ccacttgcca cctaggcgct ctggtggccc    1380
   ttgcagaagc cctaagtacg gttacttatg cgtgtagaca ttttatgtca cttattaagc    1440
   cgctggcacg gccctgcgta gcagcaccag ccggccccac ccctgctcgc ccctatcgct    1500
   ccagccaagg cgaagaagca cgaacgaatg tcgagagggg gtgaagacat ttctcaactt    1560
   ctcggccgga gtttggctga gatcgcggta ttaaatctgt gaaagaaaac aaaacaaaac    1620
   aaaaaaaaaa aaaaaaaa                                                  1638
<210> 10
   <211> 417
   <212> PRT
   <213> Homo sapiens
<400> 10
   Met Glu Gln Arg Pro Arg Gly Cys Ala Ala Val Ala Ala Ala Leu Leu
    1               5                  10                  15
   Leu Val Leu Leu Gly Ala Arg Ala Gln Gly Gly Thr Arg Ser Pro Arg
               20                  25                  30
   Cys Asp Cys Ala Gly Asp Phe His Lys Lys Ile Gly Leu Phe Cys Cys
           35                  40                  45
   Arg Gly Cys Pro Ala Gly His Tyr Leu Lys Ala Pro Cys Thr Glu Pro
       50                  55                  60
   Cys Gly Asn Ser Thr Cys Leu Val Cys Pro Gln Asp Thr Phe Leu Ala
   65                  70                  75                  80
   Trp Glu Asn His His Asn Ser Glu Cys Ala Arg Cys Gln Ala Cys Asp
                   85                  90                  95
   Glu Gln Ala Ser Gln Val Ala Leu Glu Asn Cys Ser Ala Val Ala Asp
               100                 105                 110
   Thr Arg Cys Gly Cys Lys Pro Gly Trp Phe Val Glu Cys Gln Val Ser
           115                 120                 125
   Gln Cys Val Ser Ser Ser Pro Phe Tyr Cys Gln Pro Cys Leu Asp Cys
       130                 135                 140
   Gly Ala Leu His Arg His Thr Arg Leu Leu Cys Ser Arg Arg Asp Thr
   145                 150                 155                 160
   Asp Cys Gly Thr Cys Leu Pro Gly Phe Tyr Glu His Gly Asp Gly Cys
                   165                 170                 175
   Val Ser Cys Pro Thr Ser Thr Leu Gly Ser Cys Pro Glu Arg Cys Ala
               180                 185                 190
   Ala Val Cys Gly Trp Arg Gln Met Phe Trp Val Gln Val Leu Leu Ala
           195                 200                 205
   Gly Leu Val Val Pro Leu Leu Leu Gly Ala Thr Leu Thr Tyr Thr Tyr
       210                 215                 220
   Arg His Cys Trp Pro His Lys Pro Leu Val Thr Ala Asp Glu Ala Gly
   225                 230                 235                 240
   Met Glu Ala Leu Thr Pro Pro Pro Ala Thr His Leu Ser Pro Leu Asp
                   245                 250                 255
   Ser Ala His Thr Leu Leu Ala Pro Pro Asp Ser Ser Glu Lys Ile Cys
               260                 265                 270
   Thr Val Gln Leu Val Gly Asn Ser Trp Thr Pro Gly Tyr Pro Glu Thr
           275                 280                 285
   Gln Glu Ala Leu Cys Pro Gln Val Thr Trp Ser Trp Asp Gln Leu Pro
       290                 295                 300
   Ser Arg Ala Leu Gly Pro Ala Ala Ala Pro Thr Leu Ser Pro Glu Ser
   305                 310                 315                 320
   Pro Ala Gly Ser Pro Ala Met Met Leu Gln Pro Gly Pro Gln Leu Tyr
                   325                 330                 335
   Asp Val Met Asp Ala Val Pro Ala Arg Arg Trp Lys Glu Phe Val Arg
               340                 345                 350
   Thr Leu Gly Leu Arg Glu Ala Glu Ile Glu Ala Val Glu Val Glu Ile
           355                 360                 365
   Gly Arg Phe Arg Asp Gln Gln Tyr Glu Met Leu Lys Arg Trp Arg Gln
       370                 375                 380
   Gln Gln Pro Ala Gly Leu Gly Ala Val Tyr Ala Ala Leu Glu Arg Met
   385                 390                 395                 400
   Gly Leu Asp Gly Cys Val Glu Asp Leu Arg Ser Arg Leu Gln Arg Gly
                   405                 410                 415
   Pro